# EUROPEAN PATENT SPECIFICATION

(11) **EP 1 100 530 B1**
(45) Date of publication and mention of the grant of the patent: **08.10.2003**
(21) Application number: 99934520.0
(22) Date of filing: 29.07.1999
(51) Int. Cl.: A61K 38/26, A61P 3/10

(54) **IN-VITRO STIMULATION OF BETA CELL PROLIFERATION**
IN-VITRO STIMULATION VON BETA ZELLEN VERMEHRUNG
IN VITRO STIMULATION DE LA PROLIFERATION DES CELLULES DE LANGERHANS BETA PANCREATIQUE

(30) Priority: 31.07.1998 DK 99898; 12.08.1998 DK 102598
(43) Date of publication of application: 23.05.2001
(60) Divisional application: 02026139.2
(73) Proprietor: NOVO NORDISK A/S, 2880 Bagsvaerd (DK)
(72) Inventor: NIELSEN, Jens, H iriis, DK-2100 Copenhagen (DK); FRIEDRICHSEN, Birgitte, Nissen, DK-2820 Gentofte (DK); RUGH, Susanne, DK-2880 Bagsvaerd (DK); TROMHOLT, Niels, DK-2920 Charlottenlund (DK); BJORN, Soren, DK-2800 Lyngby (DK); KNUDSEN, Liselotte, Bjerre, DK-2500 Valby (DK); STURIS, Jeppe, DK-3500 Vaerloese (DK)
(74) Representative: Kjerrumgaard, Lars
(86) International application number: DK9900424
(87) International publication number: WO00007617

(56) References cited:
- WO-A1-98/08871
- JENS J. HOLST ET AL: 'Perspective in Diabetes. Inhibition of the Activity of Dipeptidyl-Peptidase IV as a Treatment for Type 2 Diabetes' DIABETES vol. 47, November 1998, pages 1663 - 1670, XP002921064
- M.M. BYRNE ET AL: 'Human Studies with Glucagon-Like-Peptide-1: Potential of the Gut Hormone for Clinical Use' DIABETIC MEDICINE vol. 13, 1996, GERMANY, pages 854 - 860, XP002921065

## Description

The present invention relates to a method for increasing the number and/or the size of beta cells, and for stimulating beta cell proliferation. The invention is based on the recognition that GLP-1 acts as a beta cell growth factor.

Diabetes is characterized by insufficiency of the pancreatic beta cells to. maintain normoglycemia. In type 1 diabetes (IDDM) this is due to destruction of the beta cells by an autoimmune process whereas in type 2 diabetes (NIDDM) it is due to a combination of beta cell deficiency and peripheral insulin resistance.. Under normal conditions the number of beta cells shows a positive correlation with the body mass. However in diabetic patients the number of beta cells is reduced and it is therefore pertinent not only to improve the function of the beta cells by therapeutical means but also to increase the number. of beta cells. GLP-1 has been shown to stimulate glucose-induced insulin release and insulin biosynthesis and to restore glucose competence, but to. our knowledge no reports on stimulation of beta cell proliferation have appeared. In our efforts to identify beta cell growth factors we discovered that GLP-1 indeed could stimulate beta cell proliferation in vitro. The proliferation was measured as incorporation of the thymidine analogue 5-bromo-2-deoxyuridine into. DNA in insulin positive cells in pancreatic islet cells from newborn rats. GLP-1 was found to increase the number of labelled beta cells.. This may have important implication for the treatment and/or prevention of diabetes.

Accordingly, the present invention relates to a method for increasing the number and/or the size of beta cells comprising stimulating beta cell proliferation in vitro using GLP-1 or an analogue or a derivative thereof or a GLP-1 agonist; a method for increasing the number of beta cells comprising stimulating beta cell proliferation in vitro using GLP-1 or an analogue or a derivative thereof or a GLP-1 agonist;. a method for increasing the size of beta cells comprising stimulating beta cell proliferation in vitro using GLP-1 or an analogue or a derivative thereof or a GLP-1 agonist; a method for stimulating beta cell proliferationcomprising the use of GLP-1 or an analogue or a derivative thereof or a GLP-1. agonist; the use of GLP-1 or an analogue or a derivative thereof or a GLP-1 agonist as a beta cell growth factor; a method for preventing Type I or Type II diabetes comprising using GLP-1 or an analogue or a derivative thereof or a GLP-1 agonist; a method for increasing c-peptide levels comprising the use of GLP-1 or an analogue or a derivative thereof or a GLP-1 agonist; a method for obtaining a less severe disease stage in a subject suffering from Type II diabetes comprising stimulating beta cell proliferation in vitro using GLP-1 or an analogue or a derivative thereof or a GLP-1 agonist; a method for increasing the insulin synthesis capability of a subject comprising stimulating beta cell proliferation in vitro using GLP-1 or an analogue or a derivative thereof or a GLP-1 agonist; a method of delaying the progression of impaired glucose tolerance (IGT) to insulin requiring Type II diabetes comprising stimulating beta cell proliferation in vitro using GLP-1. or an analogue or a derivative thereof or a GLP-1 agonist; a method of delaying the progression of non-insulin requiring Type II diabetes to insulin requiring Type II diabetes comprising stimulating beta cell proliferation in vitro using GLP-1 or an analogue or a derivative thereof or a GLP-1 agonist; a method for curing Type I or Type II diabetes comprising stimulating beta cell proliferation in vitro using GLP-1 or an analogue or a derivative thereof or a GLP-1 agonist; a method according to. any of the above methods which further comprises the use of human growth hormone, a growth hormone releasing agent or a growth factor such as prolactin or placental lactogen; a method for increasing the number and/or the size of beta cells comprising the use of human growth hormone, a growth hormone releasing agent or a growth factor such as prolactin or placental lactogen; and a method for stimulating beta cell proliferation comprising the use of human growth hormone, a growth hormone releasing agent or a growth factor such as prolactin or placental lactogen.

The subject is preferably a mammal, more preferably a human.

The invention furthermore relates to. the use of GLP-1 or an analogue or a derivative thereof or a GLP-1 agonist for the preparation of a composition for increasing the number and/or the size of beta cells; the use of GLP-1 or an analogue or a derivative thereof or a GLP-1 agonist for the preparation of a composition for increasing the number of beta cells; the use of GLP- 1 or an analogue or a derivative thereof or a GLP-1 agonist for the preparation of a composition for increasing the size of beta cells in a subject; the use of GLP-1 or an analogue or a derivative thereof or a GLP-1 agonist for the preparation of a composition for stimulating beta cell proliferation; the use of GLP-1 or an analogue or a derivative thereof or a GLP-1 agonist for stimulation of beta cell proliferation in vitro.

In the present context "GLP-1 or an analogue or a derivative thereof or a GLP-1 agonist" is also intended to comprise active metabolites and prodrugs of GLP-1 or an analogue or a derivative thereof or a GLP-1 agonist. A "metabolite" is an active derivative of GLP-1 or an analogue or a derivative thereof or a GLP-1 agonist produced when the GLP-1 or an analogue or a derivative thereof or a GLP-1 agonist is metabolized. A "prodrug" is a compound which is either metabolized to GLP-1 or an analogue or a derivative thereof or a GLP-1 agonist or is metabolized to. the same metabolite(s) as GLP-1 or an analogue or a derivative thereof or a GLP-1 agonist.

In the present context "GLP-1 agonists" is intended to indicate a molecule, preferably a non-peptide, which binds to a GLP-1 receptor with an affinity constant, K_{D}, below 1 µM, preferably below 100 nM. Methods for identifying GLP-1 agonists are described in WO 93/19175 (Novo Nordisk A/S). Examples of GLP-1 agonists to be included within the present invention are exendins as disclosed in WO 9746584 and US 5424286. US 5424286 describes a method for stimulating insulin release with exendin polypeptide(s). The exendin polypeptides disclosed include HGEGTFTSDLSKQMEEEAVRLFIEWLKNGGX; wherein X = P or Y, and HX1X2GTFITSDLSKQMEEEAVRLFIEWLKNGGPSSGAPPPS; wherein X1X2 = SD (exendin-3) or GE (exendin-4)). The exendin-3 and -4 and fragments are useful in treatment of diabetes mellitus (types I or II) and prevention of hyperglycaemia. They normalise hyperglycaemia through glucose-dependent, insulin-independent and insulin-dependent mechanisms. These insulinotropic peptides are more active than GLP-1. Exendin-4 is specific for exendin receptors, i.e. it does not interact with vasoactive intestinal peptide receptors. WO 9746584 describes truncated versions of exendin peptide(s) for treating diabetes. The disclosed peptides increase secretion and biosynthesis of insulin, but reduce those of glucagon. The truncated peptides can be made more economically than full length versions.

In the present text, the designation "an analogue" is used to designate a peptide wherein one or more amino acid residues of the parent peptide have been substituted by another amino acid residue and/or wherein one or more amino acid residues of the parent peptide have been deleted and/or wherein one or more amino. acid residues have been added to the parent peptide. Such addition can take place either in the peptide, at the N-terminal end or at the C-terminal end of the parent peptide, or any combination thereof.

The term "derivative" is used in the present text to designate a peptide in which one or more of the amino acid residues of the parent peptide have been chemically modified, *e.g*. by alkylation, acylation, ester formation or amide formation.

The term "a GLP-1 derivative" is used in the present text to designate a derivative of GLP-1 or an analogue thereof. In the present text, the parent peptide from which such a derivative is formally derived is in some places referred to as the "GLP-1 moiety" of the derivative.

### Lipophilic Substituents

In the GLP-1 derivatives of the present invention, one or more lipophilic substituents may be attached to the parent peptide. The lipophilic substituents make the profile of action of the parent GLP-1 peptide more protracted, make the parent GLP-1 peptide more metabolically and physically stable, and/or increase the water solubility of the parent GLP-1 peptide.

The lipophilic substituent is characterised by having a solubility in water at 20°C in the range from about 0.1 mg/100 ml water to about 250 mg/100 ml water, preferable in the range from about 0.3 mg/100 ml water to about 75. mg/100 ml water. For instance, octanoic acid (C8) has a solubility in water at 20°C of 68 mg/100 ml, decanoic acid (C10) has a solubility in water at 20°C of 15 mg/100 ml, and octadecanoic acid (C18) has a solubility in water at 20°C of 0.3 mg/100 ml.

The GLP-1 derivatives of the present invention preferably have three lipophilic substituents, more preferably two lipophilic substituents, and most preferably one lipophilic substituent.

Each lipophilic substituent(s) preferably has 4-40 carbon atoms, more preferably 8- ' 30 carbon atoms, even more preferably 8-25 carbon atoms, even more preferably 12-25 carbon atoms, and most preferably 14-18 carbon atoms.

The lipophilic substituent(s) contain a functional group which can be attached to one of the following functional groups of an amino acid of the parent GLP-1 peptide:
(a) the amino group attached to the alpha-carbon of the N-terminal amino acid,
(b) the carboxy group attached to the alpha-carbon of the C-terminal amino acid,
(c) the epsilon-amino group of any Lys residue,
(d) the carboxy group of the R group of any Asp and Glu residue,
(e) the hydroxy group of the R group of any Tyr, Ser and Thr residue,
(f) the amino group of the R group of any Trp, Asn, Gin, Arg, and His residue, or
(g) the thiol group of the R group of any Cys residue.

In an embodiment, a lipophilic substituent is attached to. the carboxy group of the R group of any Asp and Glu residue.

In another embodiment, a lipophilic substituent is attached to the carboxy group attached to the alpha-carbon of the C-terminal amino acid.

In a most preferred embodiment, a lipophilic substituent is attached to the epsilon-amino group of any Lys residue.

Each lipophilic substituent contains a functional group which may be attached to a functional group of an amino acid of the parent GLP-1 peptide. For example, a lipophilic substituent may contain a carboxyl group. which can be attached to an amino group of the parent GLP-1 peptide by means of an amide bond.

In an embodiment, the lipophilic substituent comprises a partially or completely hydrogenated cyclopentanophenathrene skeleton.

In another embodiment, the lipophilic substituent is a straight-chain or branched alkyl group.

In another embodiment, the lipophilic substituent is an acyl group of a straight-chain or branched fatty acid. Preferably, the lipophilic substituent is an acyl group having the formula CH₃(CH₂)ₙCO-, wherein n is an integer from 4 to 38, preferably an integer from 12 to 38, and most preferably is CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-. In a more preferred embodiment, the lipophilic substituent is tetradecanoyl. In a most preferred embodiment, the lipophilic substituent is hexadecanoyl.

In another embodiment of the present invention, the lipophilic substituent has a group which is negatively charged such as a carboxylic acid group. For example, the lipophilic substituent may be an acyl group of a straight-chain or branched alkane α,ω-dicarboxylic acid of the formula HOOC(CH₂)ₘCO-, wherein m is an integer from 4 to 38, preferably an integer from 12 to 38, and most preferably is HOOC(CH₂)₁₄CO-, HOOC(CH₂)₁₆CO-, HOOC(CH₂)₁₈CO-, HOOC(CH₂)₂₀CO- or HOOC(CH₂)₂₂CO-.

In a preferred embodiment of the invention, the lipophilic substituent is attached to the parent GLP-1 peptide by means of a spacer. A spacer must contain at least two functional groups, one to attach to a functional group of the lipophilic substituent and the other to a functional group of the parent GLP-1 peptide.

In an embodiment, the spacer is an amino acid residue except Cys or Met, or a dipeptide such as Gly-Lys. For purposes of the present invention, the phrase "a dipeptide such as Gly-Lys" means any combination of two amino acids except Cys or Met, preferably a dipeptide wherein the C-terminal amino acid residue is Lys, His or Trp, preferably Lys, and the N-terminal amino acid residue is Ala, Arg, Asp, Asn, Gly, Glu, Gln, Ile, Leu, Val, Phe, Pro, Ser, Tyr, Thr, Lys, His and Trp. Preferably, an amino group of the parent peptide. forms an amide bond with a carboxylic group of the amino acid residue or dipeptide spacer, and an amino group of the amino acid residue or dipeptide spacer forms an amide bond with a carboxyl group of the lipophilic substituent.

Preferred spacers are lysyl, glutamyl, asparagyl, glycyl, beta-alanyl and gamma-aminobutanoyl, each of which constitutes an individual embodiment. Most preferred spacers are glutamyl and beta-alanyl. When the spacer is Lys, Glu or Asp, the carboxyl group thereof may form an amide bond with an amino group of the amino. acid residue, and the amino group thereof may form an amide bond with a carboxyl group of the lipophilic substituent. When Lys is used as the spacer, a further spacer may in some instances be inserted between the ε-amino group of Lys and the lipophilic substituent. In one embodiment, such a further spacer is succinic acid which forms an amide bond with the ε-amino group of Lys and with an amino group present in the lipophilic substituent. In another embodiment such a further spacer is Glu or Asp which forms an amide bond with the ε-amino group of Lys and another amide bond with a carboxyl group present in the lipophilic substituent, that is, the lipophilic substituent is a N^{ε}-acylated lysine residue.

In another embodiment, the spacer is an unbranched alkane α,ω-dicarboxylic acid group having from 1 to 7 methylene groups, which spacer forms a bridge between an amino group of the parent peptide and an amino group of the lipophilic substituent. Preferably, the spacer is succinic acid.

In a further embodiment, the lipophilic substituent with the attached spacer is a group of the formula CH₃(CH₂)ₚNH-CO(CH₂)_{q}CO-, wherein p is an integer from 8 to 33, preferably from 12 to 28 and q is an integer from 1. to 6, preferably 2.

In a further embodiment, the lipophilic substituent with the attached spacer is a group of the formula CH₃(CH₂)ᵣCO-NHCH(COOH)(CH₂)₂CO-, wherein r is an integer from 4 to 24, preferably from 10 to 24.

In a further embodiment, the lipophilic substituent with the attached spacer is a group of the formula CH₃(CH₂)ₛCO-NHCH((CH₂)₂COOH)CO-, wherein s is an integer from 4 to 24, preferably from 10 to 24.

In a further embodiment, the lipophilic substituent is a group of the formula COOH(CH₂)ₜCO- wherein t is an integer from 6 to 24.

In a further embodiment, the lipophilic substituent with the attached spacer is a group of the formula -NHCH(COOH)(CH₂)₄NH-CO(CH₂)ᵤCH₃, wherein u is an integer from 8 to 18.

In a further embodiment, the lipophilic substituent with the attached spacer is a group of the formula CH₃(CH₂)ᵥCO-NH-(CH₂)_{z}-CO, wherein v is an integer from 4 to 24 and z is an integer from 1 to 6.

In a further embodiment, the lipophilic substituent with the attached spacer is a group of the formula -NHCH(COOH)(CH₂)₄NH-COCH((CH₂)₂COOH)NH-CO(CH₂)_{w}CH₃, wherein w is an integer from 10 to 16.

In a further embodiment, the lipophilic substituent with the attached spacer is a group of the formula -NHCH(COOH)(CH₂)₄NH-CO(CH₂)₂CH(COOH)NHCO(CH₂)ₓCH₃, wherein x is zero or an integer from 1. to 22, preferably 10 to 16.

The term "GLP-1" means GLP-1(7-37) or GLP-1(7-36) amide.

GLP-1 analogues and derivatives which can be used according to the present invention includes those referred to in PCT/DK99/00081 (Novo Nordisk A/S), PCT/DK99/00082 (Novo Nordisk A/S), PCT/DK99/00085 (Novo Nordisk A/S), WO 98/08871 (Novo Nordisk A/S), WO 87/06941 (The General Hospital Corporation), WO 90/11296 (The General Hospital Corporation), WO 91/11457 (Buckley et al.), EP 0708179-A2 (Eli Lilly & Co.), EP 0699686-A2 (Eli Lilly & Co.) which are included herein by reference.

In one embodiment GLP-1 or an analogue or a derivative thereof or a GLP-1 agonist is GLP-1(7-37).

In another embodiment GLP-1 or an analogue or a derivative thereof or a GLP-1 agonist is GLP-1(7-36) amide.

In a further embodiment GLP-1 or an analogue or a derivative thereof or a GLP-1 agonist is an analogue of GLP-1.

In a further embodiment the analogue of GLP-1 has the formula II: 
7 8 9 10 11 12 13 14 15 16 17
His-Xaa-Xaa-Gly-Xaa-Phe-Thr-Xaa-Asp-Xaa-Xaa-

18 19 20 21 22 23 24 25 26 27 28
Xaa-Xaa-Xaa-Xaa-Xaa-Xaa-Xaa-Xaa-Xaa-Xaa-Phe-

29 30 31 32 33 34 35 36 37 38
Ile-Xaa-Xaa-Xaa-Xaa-Xaa-Xaa-Xaa-Xaa-Xaa

39 40 41 42 43 44 45
Xaa-Xaa-Xaa-Xaa-Xaa-Xaa-Xaa

(II)
 wherein
Xaa at position 8 is Ala, Gly, Ser, Thr, Leu, Ile, Val, Glu, Asp, Met, or Lys,
Xaa at position 9 is Glu, Asp, or Lys,
Xaa at position 11 is Thr, Ala, Gly, Ser, Leu, Ile, Val, Glu, Asp, or Lys,
Xaa at position 14 is Ser, Ala, Gly, Thr, Leu, Ile, Val, Glu, Asp, or Lys,
Xaa at position 16 is Val, Ala, Gly, Ser, Thr, Leu, Ile, Tyr, Glu, Asp, or Lys,
Xaa at position 17 is Ser, Ala, Gly, Thr, Leu, Ile, Val, Glu, Asp, or Lys,
Xaa at position 18 is Ser, Ala, Gly, Thr, Leu, Ile, Val, Glu, Asp, or Lys,
Xaa at position 19 is Tyr, Phe, Trp, Glu, Asp, or Lys,
Xaa at position 20 is Leu, Ala, Gly, Ser, Thr, Leu, De, Val, Glu, Asp, or Lys,
Xaa at position 21 is Glu, Asp, or Lys,
Xaa at position 22 is Gly, Ala, Ser, Thr, Leu, Ile, Val, Glu, Asp, or Lys,
Xaa at position 23 is Gln, Asn, Arg, Glu, Asp, or Lys,
Xaa at position 24 is Ala, Gly, Ser, Thr, Leu, De, Val, Arg, Glu, Asp, or Lys,
Xaa at position 25 is Ala, Gly, Ser, Thr, Leu, Ile, Val, Glu, Asp, or Lys,
Xaa at position 26 is Lys, Arg, Gln, Glu, Asp, or His,
Xaa at position 27 is Glu, Asp, or Lys,
Xaa at position 30 is Ala, Gly, Ser, Thr, Leu, Ile, Val, Glu, Asp, or Lys,
Xaa at position 31 is Trp, Phe, Tyr, Glu, Asp, or Lys,
Xaa at position 32 is Leu, Gly, Ala, Ser, Thr, Ile, Val, Glu, Asp, or Lys,
Xaa at position 33. is Val, Gly, Ala, Ser, Thr, Leu, Ile, Glu, Asp, or Lys,
Xaa at position 34 is Lys, Arg, Glu, Asp, or His,
Xaa at position 35 is Gly, Ala, Ser, Thr, Leu, Ile, Val, Glu, Asp, or Lys,
Xaa at position 36 is Arg, Lys, Glu, Asp, or His,
Xaa at position 37 is Gly, Ala, Ser, Thr, Leu, De, Val, Glu, Asp, or Lys, or is deleted,
Xaa at position 38 is Arg, Lys, Glu, Asp, or His, or is deleted,
Xaa at position 39 is Arg, Lys, Glu, Asp, or His, or is deleted,
Xaa at position 40 is Asp, Glu, or Lys, or is deleted,
Xaa at position 41. is Phe, Trp, Tyr, Glu, Asp, or Lys, or is deleted,
Xaa at position 42 is Pro, Lys, Glu, or Asp, or is deleted,
Xaa at position 43 is Glu, Asp, or Lys, or is deleted,
Xaa at position 44 is Glu, Asp, or Lys, or is deleted, and
Xaa at position 45 is Val, Glu, Asp, or Lys, or is deleted, or
   (a) a C-1-6-ester thereof, (b) amide, C-1-6-alkylamide, or C-1-6-dialkylamide thereof and/or (c) a pharmaceutically acceptable salt thereof,
provided that
(i) when the amino acid at position 37, 38, 39, 40, 41, 42, 43 or 44 is deleted, then each amino acid downstream of the amino acid is also deleted.

In a further embodiment of the GLP-1 analogue of formula II, the amino. acids at positions 37-45 are absent.

In another. embodiment of the GLP-1 analogue of formula II, the amino acids at positions 38-45 are absent

In another embodiment of the GLP-1 analogue of formula II, the amino acids at positions 39-45 are absent.

In another embodiment of the GLP-1 analogue of formula II, Xaa at position 8 is Ala, Gly, Ser, Thr, Met, or Val.

In another embodiment of the GLP-1 analogue of formula II, Xaa at position 8 is Gly, Thr, Met, or Val.

In another embodiment of the GLP-1 analogue of formula II, Xaa at position 8 is Val.

In another embodiment of the GLP-1 analogue of formula II, Xaa at position 9 is Glu.

In another embodiment of the GLP-1 analogue of formula II, Xaa at position 11 is Thr.

In another embodiment of the GLP-1 analogue of formula II, Xaa at position 14 is Ser.

In another embodiment of the GLP-1 analogue of formula II, Xaa at position 16 is Val.

In another embodiment of the GLP-1 analogue of formula II, Xaa at position 17 is Ser.

In another embodiment of the GLP-1 analogue of formula II, Xaa at position 18 is Ser, Lys, Glu, or Asp.

In another embodiment of the GLP-1. analogue of formula II, Xaa at position 19 is Tyr, Lys, Glu, or Asp.

In another embodiment of the GLP-1 analogue of formula II, Xaa at position 20 is Leu, Lys, Glu, or Asp.

In another embodiment of the GLP-1 analogue of formula II, Xaa at position 21 is Glu, Lys, or Asp.

In another embodiment of the GLP-1 analogue of formula II, Xaa at position 22 is Gly, Glu, Asp, or Lys.

In another embodiment of the GLP-1 analogue of formula II, Xaa at position 23 is Gln, Glu, Asp, or Lys.

In another embodiment of the GLP-1 analogue of formula II, Xaa at position 24 is Ala, Glu, Asp, or Lys.

In another embodiment of the GLP-1 analogue of formula II, Xaa at position 25 is Ala, Glu, Asp, or Lys.

In another embodiment of the GLP-1 analogue of formula II, Xaa at position 26 is Lys, Glu, Asp, or Arg.

In another embodiment of the GLP-1 analogue of formula II, Xaa at position 27 is Glu, Asp, or Lys.

In another embodiment of the GLP-1 analogue of formula II, Xaa at position 30 is Ala, Glu, Asp, or Lys.

In another embodiment of the GLP-1 analogue of formula II, Xaa at position 31 is Trp, Glu, Asp, or Lys.

In another embodiment of the GLP-1 analogue of formula II, Xaa at position 32 is Leu, Glu, Asp, or Lys.

In another embodiment of the GLP-1 analogue of formula II, Xaa at position 33. is Val, Glu, Asp, or Lys.

In another embodiment of the GLP-1 analogue of formula II, Xaa at position 34 is Lys, Arg, Glu, or Asp.

In another embodiment of the GLP-1 analogue of formula II, Xaa at position 35 is Gly, Glu, Asp, or Lys.

In another embodiment of the GLP-1 analogue of formula II, Xaa at position 36 is Arg, Lys, Glu, or Asp.

In another embodiment of the GLP-1 analogue of formula II, Xaa at position 37 is Gly, Glu, Asp, or Lys.

In another embodiment of the GLP-1 analogue of formula II, Xaa at position 38 is Arg, or Lys, or is deleted.

In another embodiment of the GLP-1 analogue of formula II, Xaa at position 39 is deleted.

In another embodiment of the GLP-1 analogue of formula II, Xaa at position 40 is deleted.

In another embodiment of the GLP-1 analogue of formula II, Xaa at position 41 is deleted.

In another embodiment of the GLP-1 analogue of formula II, Xaa at position 42 is deleted.

In another embodiment of the GLP-1 analogue of formula II, Xaa at position 43 is deleted.

In another embodiment of the GLP-1 analogue of formula II, Xaa at position 44 is deleted.

In another embodiment of the GLP-1 analogue of formula II, Xaa at position 45. is deleted.

In another embodiment of the GLP-1 analogue of formula II, Xaa at position 26 is Arg, each of Xaa at positions 37-45 is deleted, and each of the other Xaa is the amino acid in native GLP-1(7-36).

In another embodiment of the GLP-1 analogue of formula II, Xaa at position 26 is Arg, each of Xaa at positions 38-45 is deleted, and each of the other Xaa is the amino. acid in native GLP-1(7-37).

In another embodiment of the GLP-1 analogue of formula II, Xaa at position 26 is Arg, each of Xaa at positions 39-45 is deleted, and each of the other Xaa is the amino acid in native GLP-1(7-38).

In another embodiment of the GLP-1 analogue of formula II, Xaa at position 34 is Arg, each of Xaa at positions 37-45 is deleted, and each of the other Xaa is the amino acid in native GLP-1(7-36).

In another embodiment of the GLP-1 analogue of formula II, Xaa at position 34 is Arg, each of Xaa at positions 38-45 is deleted, and each of the other Xaa is the amino acid in native GLP-1(7-37).

In another embodiment of the GLP-1 analogue of formula II, Xaa at position 34 is Arg, each of Xaa at positions 39-45 is deleted, and each of the other Xaa is the amino acid in native GLP-1(7-38).

In another embodiment of the GLP-1 analogue of formula II, Xaa at positions 26 and 34 is Arg, Xaa at position 36 is Lys, each of Xaa at positions 37-45 is deleted, and each of the other Xaa is the amino acid in native GLP-1(7-36).

In another embodiment of the GLP-1 analogue of formula II, Xaa at positions 26 and 34 is Arg, Xaa at position 36 is Lys, each of Xaa at positions 38-45 is deleted, and each of the other Xaa is the amino acid in native GLP-1(7-37).

In another embodiment of the GLP-1 analogue of formula II, Xaa at positions 26 and 34 is Arg, Xaa at position 36 is Lys, each of Xaa at positions 39-45 is deleted, and each of the other Xaa is the amino acid in native GLP-1(7-38).

In another embodiment of the GLP-1 analogue of formula II, Xaa at positions 26 and 34 is Arg, Xaa at position 38 is Lys, each of Xaa at positions 39-45 is deleted, and each of the other Xaa is the amino acid in native GLP-1(7-38).

In another embodiment of the GLP-1 analogue of formula II, Xaa at position 8 is Thr, Ser, Gly, or Val, Xaa at position 37 is Glu, Xaa at position 36 is Lys, each of Xaa at positions 38-45 is deleted, and each of the other Xaa is the amino acid in native GLP-1(7-37).

In another embodiment of the GLP-1 analogue of formula II, Xaa at position 8 is Thr, Ser, Gly, or Val, Xaa at position 37 is Glu, Xaa at position 36 is Lys, each of Xaa at positions 39-45 is deleted, and each of the other Xaa is the amino acid in native GLP-1(7-38).

In another embodiment of the GLP-1 analogue of formula II, Xaa at position 8 is Thr, Ser, Gly or Val, Xaa at position 37 is Glu, Xaa at position 38 is Lys, each of Xaa at positions 39-45 is deleted, and each of the other Xaa is the amino acid in native GLP-1(7-38).

In another embodiment of the GLP-1 analogue of formula II, Xaa at position 18, 23 or 27 is Lys, and Xaa at positions 26 and 34 is Arg, each of Xaa at positions 37-45 is deleted, and each of the other Xaa is the amino acid in native GLP-1(7-36).

In another embodiment of the GLP-1 analogue of formula II, Xaa at position 18, 23 or 27 is Lys, and Xaa at positions 26 and 34. is Arg, each of Xaa at positions 38-45 is deleted, and each of the other Xaa is the amino acid in native GLP-1(7-37).

In another embodiment of the GLP-1 analogue of formula II, Xaa at position 18, 23 or 27 is Lys, and Xaa at positions 26 and 34 is Arg, each of Xaa at positions 39-45 is deleted, and each of the other Xaa is the amino acid in native GLP-1(7-38).

In another embodiment of the GLP-1 analogue of formula II, Xaa at position 8 is Thr, Ser, Gly, or Val, Xaa at position 18, 23 or 27 is Lys, and Xaa at position 26 and 34 is Arg, each of Xaa at positions 37-45 is deleted, and each of the other Xaa is the amino acid in native GLP-1(7-36).

In another embodiment of the GLP-1. analogue of formula II, Xaa at position 8 is Thr, Ser, Gly, or Val, Xaa at position 18, 23 or 27 is Lys, and Xaa at position 26 and 34 is Arg, each of Xaa at positions 38-45 is deleted, and each of the other Xaa is the amino acid in native GLP-1(7-37).

In another embodiment of the GLP-1 analogue of formula II, Xaa at position 8 is Thr, Ser, Gly, or Val, Xaa at position 18, 23 or 27 is Lys, and Xaa at position 26 and 34 is Arg, each of Xaa at positions 39-45 is deleted, and each of the other Xaa is the amino acid in native GLP-1(7-38).

Such GLP-1 analogues includes, but is not limited to, Arg²⁶-GLP-1(7-37); Arg³⁴-GLP-1(7-37); Lys³⁶-GLP-1(7-37); Arg^{26,34}Lys³⁶-GLP-1(7-37); Arg^{26,34}Lys³⁸GLP-1(7-38); Arg^{26,34}Lys³⁹-GLP-1(7-39); Arg^{26,34}Lys⁴⁰-GLP-1(7-40); Arg²⁶Lys³⁶-GLP-1(7-37); Arg³⁴Lys³⁶-GLP-1(7-37); Arg²⁶Lys³⁹-GLP-1(7-39); Arg³⁴Lys⁴⁰-GLP-1(7-40); Arg^{26,34}Lys^{36,39}-GLP-1(7-39); Arg^{26,34}Lys^{36,40}-GLP-1(7-40); Gly⁸Arg²⁶-GLP-1(7-37); Gly⁸Arg³⁴-GLP-1(7-37); Val⁸-GLP-1(7-37); Thr⁸-GLP-1(7-37); Gly⁸-GLP-1(7-37); Met⁸-GLP-1(7-37); Gly⁸Lys³⁶-GLP-1(7-37); Gly⁸Arg^{26,34}Lys³⁶-GLP-1(7-37); Gly⁸Arg^{26,34}Lys³⁹-GLP-1(7-39); Gly⁸Arg^{26,34}Lys⁴⁰-GLP-1(7-40); Gly⁸Arg²⁶Lys³⁶-GLP-1(7-37); Gly⁸Arg³⁴Lys³⁶-GLP-1(7-37); Gly⁸Arg²⁶Lys³⁹-GLP-1(7-39); Gly⁸Arg³⁴Lys⁴⁰-GLP-1(7-40); Gly⁸Arg^{26,34}Lys^{36,39}-GLP-1(7-39); Gly⁸Arg^{26,34}Lys^{36,40}-GLP-1(7-40); Arg^{26,34}Lys³⁸GLP-1(7-38); Arg^{26,34}Lys³⁹GLP-1(7-39); Arg^{26,34}Lys⁴⁰GLP-1(7-40); Arg^{26,34}Lys⁴¹GLP-1(7-41); Arg^{26,34}Lys⁴²GLP-1(7-42); Arg^{26,34}Lys⁴³GLP-1(7-43); Arg^{26,34}Lys⁴⁴GLP-1(7-44); Arg^{26,34}Lys⁴⁵GLP-1(7-45); Arg^{26,34}Lys³⁸GLP-1(1-38); Arg^{26,34}Lys³⁹GLP-1(1-39); Arg^{26,34}Lys⁴⁰GLP-1(1-40); Arg^{26,34}Lys⁴¹GLP-1(1-41); Arg^{26,34}Lys⁴²GLP-1(1-42); Arg^{26,34}Lys⁴³GLP-1(1-43); Arg^{26,34}Lys⁴⁴GLP-1(1-44); Arg^{26,34}Lys⁴⁵GLP-1(1-45); Arg^{26,34}Lys³⁸GLP-1(2-38); Arg^{26,34}Lys³⁹GLP-1(2-39); Arg^{26,34}Lys⁴⁰GLP-1(2-40); Arg^{26,34}Lys⁴¹GLP-1(2-41); Arg^{26,34}Lys⁴²GLY-1(2-42); Arg^{26,34}Lys⁴³GLP-1(2-43); Arg^{26,34}Lys⁴⁴GLP-1(2-44); Arg^{26,34}Lys⁴⁵GLP-1(2-45); Arg^{26,34}Lys³⁸GLP-1(3-38); Arg^{26,34}Lys³⁹GLP-1(3-39); Arg^{26,34}Lys⁴⁰GLP-1(3-40); Arg^{26,34}Lys⁴¹GLP-1(3-41); Arg^{26,34}Lys⁴²GLP-1(3-42); Arg^{26,34}Lys⁴³GLP-1(3-43); Arg^{26,34}Lys⁴⁴-GLP-1(3-44); Arg^{26,34}Lys⁴⁵GLP-1(3-45); Arg^{26,34}Lys³⁸GLP-1(4-38); Arg^{26,34}Lys³⁹GLP-1(4-39); Arg^{26,34}Lys⁴⁰GLP-1(4-40); Arg^{26,34}Lys⁴¹GLP-1(4-41); Arg^{26,34}Lys⁴²GLP-1(4-42); Arg^{26,34}Lys⁴³GLP-1(4-43); Arg^{26,34}Lys⁴⁴GLP-1(4-44); Arg^{26,34}Lys⁴⁵GLP-1(4-45); Arg^{26,34}Lys³⁸GLP-1(5-38); Arg^{26,34}Lys³⁹GLP-1(5-39); Arg^{26,34}Lys⁴⁰GLP-1(5-40); Arg^{26,34}Lys⁴¹GLP-1(5-41); Arg^{26,34}Lys⁴²GLP-1(5-42); Arg^{26,34}Lys⁴³GLP-1(5-43); Arg^{26,34}Lys⁴⁴GLP-1(5-44); Arg^{26,34}Lys⁴⁵GLP-1(5-45); Arg^{26,34}Lys³⁸GLP-1(6-38); Arg^{26,34}Lys³⁹GLP-1(6-39); Arg^{26,34}Lys⁴⁰GLP-1(6-40); Arg^{26,34}Lys⁴¹GLP-1(6-41); Arg^{26,34}Lys⁴²GLP-1(6-42); Arg^{26,34}Lys⁴³GLP-1(6-43); Arg^{26,34}Lys⁴⁴GLP-1(6-44); Arg^{26,34}Lys⁴⁵GLP-1(6-45); Arg²⁶Lys³⁸GLP-1(1-38); Arg³⁴Lys³⁸GLP-1(1-38); Arg^{26,34}Lys^{36,38}GLP-1(1-38); Arg²⁶Lys³⁸GLP-1(7-38); Arg³⁴Lys³⁸GLP-1(7-38); Arg^{26,34}Lys^{36,38}GLP-1(7-38); Arg^{26,34}Lys³⁸GLP-1(7-38); Arg²⁶Lys³⁹GLP-1(1-39); Arg³⁴Lys³⁹GLP-1(1-39); Arg^{26,34}Lys^{36,39}GLP-1(1-39); Arg²⁶Lys³⁹GLP-1(7-39); Arg³⁴Lys³⁹GLP-1(7-39) and Arg^{26,34}Lys^{36,39}GLP-1(7-39). Each one of these specific GLP-1 analogues constitutes an alternative embodiment of the invention..

GLP-1(7-37) and GLY-1(7-36) amide and the corresponding Thr8, Met⁸, Gly⁸ and Val⁸ analogues thereof are preferred compounds to. be used according to this invention.

GLP-1(7-37) and GLP-1(7-36) amide and the corresponding Gly⁸ and Val⁸ analogues thereof are more preferred compounds to. be used according to this invention.

Val⁸GLP-1(7-37) and Val⁸GLP-1(7-36) amide are still more preferred compounds to be used according to. this invention.

However, protracted acting GLP-1 derivatives, in particular those described in WO 98/08871 are more preferred. The most preferred GLP-1 derivatives are those in which the parent peptide has the formula GLP-1(7-C), wherein C is 36, 37, 38, 39, 40, 41, 42, 43, 44 and 45, wherein optionally a total of up to fifteen, preferably up to ten amino. acid residues have been exchanged with any α-amino acid residue which can be coded for by the genetic code, said parent peptide comprising one or two lipophilic substituents having 4 to 40 carbon atoms, preferably from 8 to 25 carbon atoms, optionally via a spacer (such as γ-Glu or β-Ala). The substituents are preferably selected from acyl groups of straight-chained or branched fatty acids.

GLP-1 analogues and derivatives that include an N-terminal imidazole group and optionally an unbranched C₆-C₁₀ acyl group attached to the lysine residue in position 34 are also. embodiments of the invention.

In a further embodiment GLP-1 or an analogue or a derivative thereofor a GLP-1 agonist is a GLP-1 derivative.

In a further embodiment of the GLP-1 derivative at least one amino acid residue of the parent peptide has a lipophilic substituent attached.

In a further embodiment of the GLP-1 derivative at least one amino. acid residue of the parent peptide has a lipophilic substituent attached with the proviso that if only one lipophilic substituent is present and this substituent is attached to the N-terminal or to the C-terminal amino acid residue of the parent peptide then this substituent is an alkyl group or a group which has an ω-carboxylic acid group.

In another embodiment the GLP-1 derivative has only one lipophilic substituent.

In another embodiment the GLP-1 derivative has only one lipophilic substituent which substituent is an alkyl group or a group which has an ω-carboxylic acid group and is attached to the N-terminal amino acid residue of the parent peptide.

In another embodiment the GLP-1 derivative has only one lipophilic substituent which substituent is an alkyl group or a group which has an ω-carboxylic acid group and is attached to. the C-terminal amino acid residue of the parent peptide.

In another embodiment the GLP-1 derivative has only one lipophilic substituent which substituent can be attached to any one amino acid residue which is not the N-terminal or C-terminal amino acid residue of the parent peptide.

In another embodiment the GLP-1 derivative has two lipophilic substituents.

In another embodiment the GLP-1. derivative has two lipophilic substituents, one being attached to the N-terminal amino acid residue while the other is attached to the C-terminal amino acid residue.

In another embodiment the GLP-1 derivative has two lipophilic substituents, one being attached to the N-terminal amino acid residue while the other is attached to an amino acid residue which is not N-terminal or the C-terminal amino acid residue.

In another embodiment the GLP-1 derivative has two lipophilic substituents, one being attached to the C-terminal amino acid residue while the other is attached to. an amino acid residue which is not the N-terminal or the C-terminal amino acid residue.

In further embodiment the GLP-1 derivative is a derivative of formula GLP-1(7-C), wherein C is selected from the group comprising 38, 39, 40, 41, 42, 43, 44 and 45 which derivative has just one lipophilic substituent which is attached to. the C-terminal amino acid residue of the parent peptide.

In a further embodiment of the GLP-1 derivative the lipophilic substituent comprises from 4 to 40 carbon atoms, more preferred from 8 to 25 carbon atoms.

In a further embodiment of the GLP-1 derivative the lipophilic substituent has a solubility in water at 20°C in the range from about 0.1 mg/100 ml water to about 250 mg/100 ml water, preferable in the range from about 0.3 mg/100 ml water to about 75 mg/100 ml water.

In a further embodiment the GLP-1 derivative has a lipophilic substituent which is attached to an amino acid residue in such a way that a carboxyl group of the lipophilic substituent forms an amide bond with an amino group of the amino acid residue.

In a further embodiment the GLP-1 derivative has a lipophilic substituent which is attached to an amino acid residue in such a way that an amino group of the lipophilic substituent forms an amide bond with a carboxyl group of the amino acid residue.

In a further embodiment the GLP-1 derivative has a lipophilic substituent which is attached to. the parent peptide by means of a spacer.

In a further embodiment the GLP-1 derivative has a lipophilic substituent, which optionally via a spacer is attached to the ε-amino group of a Lys residue contained in the parent peptide.

In a further embodiment the GLP-1 derivative has a lipophilic substituent which is attached to the parent peptide by means of a spacer which is an unbranched alkane α,ω-dicarboxylic acid group having from 1 to 7 methylene groups, preferably two methylene groups which spacer forms a bridge between an amino group of the parent peptide and an amino group of the lipophilic substituent..

In a further embodiment the GLP-1 derivative has a lipophilic substituent which is attached to the parent peptide by means of a spacer which is an amino acid residue except Cys, or a dipeptide such as Gly-Lys.

In the present text, the expression "a dipeptide such as Gly-Lys" is used to designate a dipeptide wherein the C-terminal amino acid residue is Lys, His or Trp, preferably Lys, and wherein the N-terminal amino acid residue is selected from the group comprising Ala, Arg, Asp, Asn, Gly, Glu, Gln, De, Leu, Val, Phe and Pro.

In a further embodiment the GLP-1 derivative has a lipophilic substituent which is attached to the parent peptide by means of a spacer which is an amino acid residue except Cys, or is a dipeptide such as Gly-Lys and wherein a carboxyl group of the parent peptide forms an amide bond with an amino group of a Lys residue or a dipeptide containing a Lys residue, and the other amino group of the Lys residue or a dipeptide containing a Lys residue forms an amide bond with a carboxyl group of the lipophilic substituent.

In a further embodiment the GLP-1 derivative has a lipophilic substituent which is attached to the parent peptide by means of a spacer which is an amino acid residue except Cys, or is a dipeptide such as Gly-Lys and wherein an amino. group of the parent peptide forms an amide bond with a carboxylic group of the amino acid residue or dipeptide spacer, and an amino group of the amino acid residue or dipeptide spacer forms an amide bond with a carboxyl group of the lipophilic substituent.

In a further embodiment the GLP-1 derivative has a lipophilic substituent which is attached to the parent peptide by means of a spacer which is an amino acid residue except Cys, or is a dipeptide such as Gly-Lys and wherein a carboxyl group of the parent peptide forms an amide bond with an amino group of the amino acid residue spacer or dipeptide spacer, and the carboxyl group of the amino acid residue spacer or dipeptide spacer forms an amide bond with an amino group of the lipophilic substituent.

In a further embodiment the spacer is selected from lysyl, glutamyl, asparagyl, glycyl, beta-alanyl and gamma-aminobutanoyl. Each of these spacers constitutes an individual embodiment.. Most preferred spacers are glutamyl and beta-alanyl.

In a further embodiment the GLP-1 derivative has a lipophilic substituent which is attached to the parent peptide by means of a spacer which is an amino acid residue except Cys, or is a dipeptide such as Gly-Lys, and wherein a carboxyl group of the parent peptide forms an amide bond with an amino group of a spacer which is Asp or Glu, or a dipeptide spacer containing an Asp or Glu residue, and a carboxyl group of the spacer forms an amide bond with an amino group of the lipophilic substituent.

In a further embodiment the GLP-1 derivative has a lipophilic substituent which comprises a partially or completely hydrogenated cyclopentanophenathrene skeleton.

In a further embodiment the GLP-1 derivative has a lipophilic substituent which is a straight-chain or branched alkyl group.

In a further embodiment the GLP-1 derivative has a lipophilic substituent which is the acyl group of a straight-chain or branched fatty acid.

In a further embodiment the GLP-1 derivative has a lipophilic substituent which is an acyl group selected from the group comprising CH₃(CH₂)ₙCO-, wherein n is an integer. from 4 to 38, preferably an integer from 4 to 24, more preferred selected from the group comprising CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-.

In a further embodiment the GLP-1. derivative has a lipophilic substituent which is an acyl group of a straight-chain or branched alkane α,ω-dicarboxylic acid.

In a further embodiment the GLP-1 derivative has a lipophilic substituent which is an acyl group selected from the group comprising HOOC(CH₂)ₘCO-, wherein m is an integer from 4 to 38, preferably an integer from 4 to 24, more preferred selected from the group comprising HOOC(CH₂)₁₄CO-, HOOC(CH₂)₁₆CO-, HOOC(CH₂)₁₈CO-, HOOC(CH₂)₂₀CO- and HOOC(CH₂)₂₂CO-.

In a further embodiment the GLP-1 derivative has a lipophilic substituent which is a group of the formula CH₃(CH₂)ₚ((CH₂)_{q}COOH)CHNH-CO(CH₂)₂CO-, wherein p and q are integers and p+q is an integer of from 8 to 33, preferably from 12 to 28.

In a further embodiment the GLP-1 derivative has a lipophilic substituent which is a group of the formula CH₃(CH₂)ᵣCO-NHCH(COOH)(CH₂)₂CO-, wherein r is an integer of from 10 to 24.

In a further embodiment the GLP-1 derivative has a lipophilic substituent which is a group of the formula CH₃(CH₂)ₛCO-NHCH((CH₂)₂COOH)CO-, wherein s is an integer of from 8 to 24.

In a further embodiment the GLP-1 derivative has a lipophilic substituent which is a group of the formula COOH(CH₂)ₜCO- wherein t is an integer of from 8 to 24.

In a further embodiment the GLP-1 derivative has a lipophilic substituent which is a group. of the formula -NHCH(COOH)(CH₂)₄NH-CO(CH₂)ᵤCH₃, wherein u is an integer of from 8 to 18.

In a further embodiment the GLP-1 derivative has a lipophilic substituent which is a group of the formula -NHCH(COOH)(CH₂)₄NH-COCH((CH₂)₂COOH)NH-CO(CH₂)_{w}CH₃, wherein w is an integer of from 10 to 16.

In a further embodiment the GLP-1 derivative has a lipophilic substituent which is a group of the formula -NHCH(COOH)(CH₂)₄NH-CO(CH₂)₂CH(COOH)NH-CO(CH₂)ₓCH₃, wherein x is an integer of from 10 to 16.

In a further embodiment the GLP-1 derivative has a lipophilic substituent which is a group of the formula -NHCH(COOH)(CH₂)₄NH-CO(CH₂)₂CH(COOH)NHCO(CH₂)_{y}CH₃, wherein y is zero. or an integer of from 1 to 22.

In a further embodiment the GLP-1 derivative has a lipophilic substituent which can be negatively charged. Such a lipophilic substituent can for example be a substituent which has a carboxyl group.

In a further embodiment of the GLP-1 derivative the parent peptide is selected from the group comprising GLP-1(1-45) or an analogue thereof.

In a further embodiment the GLP-1 derivative is derived from a GLP-1 fragment selected from the group comprising GLP-1(7-35), GLP-1(7-36), GLP-1(7-36)amide, GLP-1(7-37), GLP-1(7-38), GLP-1(7-39), GLP-1(7-40) and GLP-1(7-41) or an analogue thereof.

In a further embodiment the GLP-1 analogue is derived from a GLP-1 analogue selected from the group comprising GLP-1(1-35), GLP-1(1-36), GLP-1(1-36)amide, GLP-1(1-37), GLP-1(1-38), GLP-1(1-39), GLP-1(1-40) and GLP-1(1-41) or an analogue thereof.

In a further embodiment of the GLP-1 derivative the designation analogue comprises derivatives wherein a total of up to fifteen, preferably up to ten amino acid residues have been exchanged with any α-amino acid residue.

In a further embodiment of the GLP-1 derivative the designation analogue comprises derivatives wherein a total of up to fifteen, preferably up to ten amino acid residues have been exchanged with any α-amino acid residue which can be coded for by the genetic code.

In a further embodiment of the GLP-1 derivative the designation analogue comprises derivatives wherein a total of up to. six amino acid residues have been exchanged with another α-amino acid residue which can be coded for by the genetic code.

In a further embodiment the GLP-1 derivative is a derivative of formula GLP-1(A-B) derivative wherein A is an integer from 1 to 7 and B is an integer from 38 to 45 or an analogue thereof comprising one lipophilic substituent attached to the C-terminal amino acid residue and, optionally, a second lipophilic. substituent attached to one of the other amino acid residues.

In a further embodiment the GLP-1 derivative is a GLP-1 derivative of formula I: 
7 8 9 10 11 12 13 14 15 16 17
His-Xaa-Xaa-Gly-Xaa-Phe-Thr-Xaa-Asp-Xaa-Xaa-
 
18 19 20 21 22 23 24 25 26 27 28
Xaa-Xaa-Xaa-Xaa-Xaa-Xaa-Xaa-Xaa-Xaa-Xaa-Phe-

29 30 31 32 33 34 35 36 37 38
Ile-Xaa-Xaa-Xaa-Xaa-Xaa-Xaa-Xaa-Xaa-Xaa

39 40 41 42 43 44 45
Xaa-Xaa-Xaa-Xaa-Xaa-Xaa-Xaa

(I)
 wherein
Xaa at position 8 is Ala, Gly, Ser, Thr, Leu, Ile, Val, Glu, Asp, Met, or Lys,
Xaa at position 9 is Glu, Asp, or Lys,
Xaa at position 11 is Thr, Ala, Gly, Ser, Leu, Ile, Val, Glu, Asp, or Lys,
Xaa at position 14 is Ser, Ala, Gly, Thr, Leu, Ile, Val, Glu, Asp, or Lys,
Xaa at position 16 is Val, Ala, Gly, Ser, Thr, Leu, Ile, Tyr, Glu, Asp, or Lys,
Xaa at position 17 is Ser, Ala, Gly, Thr, Leu, Ile, Val, Glu, Asp, or Lys,
Xaa at position 18 is Ser, Ala, Gly, Thr, Leu, Ile, Val, Glu, Asp, or Lys,
Xaa at position 19 is Tyr, Phe, Trp, Glu, Asp, or Lys,
Xaa at position 20 is Leu, Ala, Gly, Ser, Thr, Leu, Ile, Val, Glu, Asp, or Lys,
Xaa at position 21 is Glu, Asp, or Lys,
Xaa at position 22 is Gly, Ala, Ser, Thr, Leu, Ile, Val, Glu, Asp, or Lys,
Xaa at position 23 is Gln, Asn, Arg, Glu, Asp, or Lys,
Xaa at position 24 is Ala, Gly, Ser, Thr, Leu, Ile, Val, Arg, Glu, Asp, or Lys,
Xaa at position 25 is Ala, Gly, Ser, Thr, Leu, Ile, Val, Glu, Asp, or Lys,
Xaa at position 26 is Lys, Arg, Gln, Glu, Asp, or His,
Xaa at position 27 is Glu, Asp, or Lys,
Xaa at position 30 is Ala, Gly, Ser, Thr, Leu, Ile, Val, Glu, Asp, or Lys,
Xaa at position 31 is Trp, Phe, Tyr, Glu, Asp, or Lys,
Xaa at position 32 is Leu, Gly, Ala, Ser, Thr, Ile, Val, Glu, Asp, or Lys,
Xaa at position 33 is Val, Gly, Ala, Ser, Thr, Leu, Ile, Glu, Asp, or Lys,
Xaa at position 34 is Lys, Arg, Glu, Asp, or His,
Xaa at position 35 is Gly, Ala, Ser, Thr, Leu, Ile, Val, Glu, Asp, or Lys,
Xaa at position 36 is Arg, Lys, Glu, Asp, or His,
Xaa at position 37 is Gly, Ala, Ser, Thr, Leu, Ile, Val, Glu, Asp, or Lys, or is deleted,
Xaa at position 38 is Arg, Lys, Glu, Asp, or His, or is deleted,
Xaa at position 39 is Arg, Lys, Glu, Asp, or His, or is deleted,
Xaa at position 40 is Asp, Glu, or Lys, or is deleted,
Xaa at position 41 is Phe, Trp, Tyr, Glu, Asp, or Lys, or is deleted,
Xaa at position 42 is Pro, Lys, Glu, or Asp, or is deleted,
Xaa at position 43 is Glu, Asp, or Lys, or is deleted,
Xaa at position 44 is Glu, Asp, or Lys, or is deleted, and
Xaa at position 45 is Val, Glu, Asp, or Lys, or is deleted, or
(a) a C-1-6-ester thereof, (b) amide, C-1-6-alkylamide, or C-1-6-dialkylamide thereof and/or (c) a pharmaceutically acceptable salt thereof,
provided that
(i) when the amino acid at position 37, 38, 39, 40, 41, 42, 43 or 44 is deleted, then each amino acid downstream of the amino acid is also deleted,
(ii) the derivative of the GLP-1 analog contains only one or two Lys,
(iii) the ε-amino group of one or both Lys is substituted with a lipophilic substituent optionally via a spacer,
(iv) the total number of different amino acids between the derivative of the GLP-1 analog and the corresponding native form of GLP-1 does not exceed six.

In a further embodiment of the GLP-1 derivative of formula I, the amino acids at positions 37-45 are absent.

In another embodiment of the GLP-1 derivative of formula I, the amino acids at positions 38-45 are absent.

In another embodiment of the GLP-1 derivative of formula I, the amino acids at positions 39-45 are absent.

In another embodiment of the GLP-1 derivative of formula I, Xaa at position 8 is Ala, Gly, Ser, Thr, Leu, Ile, Val, Glu, Asp, or Lys,

In another embodiment of the GLP-1 derivative of formula I, Xaa at position 8 is Ala, Gly, Ser, Thr, or Val.

In another embodiment of the GLP-1 derivative of formula I, Xaa at position 9 is Glu.

In another embodiment of the GLP-1 derivative of formula I, Xaa at position 11 is Thr.

In another embodiment of the GLP-1 derivative of formula I, Xaa at position 14 is Ser.

In another embodiment of the GLP-1 derivative of formula I, Xaa at position 16 is Val.

In another embodiment of the GLP-1 derivative of formula I, Xaa at position 17 is Ser.

In another embodiment of the GLP-1 derivative of formula I, Xaa at position 18 is Ser, Lys, Glu, or Asp.

In another embodiment of the GLP-1 derivative of formula I, Xaa at position 19 is Tyr, Lys, Glu, or Asp.

In another embodiment of the GLP-1 derivative of formula I, Xaa at position 20 is Leu, Lys, Glu, or Asp.

In another embodiment of the GLP-1 derivative of formula I, Xaa at position 21 is Glu, Lys, or Asp.

In another embodiment of the GLP-1 derivative of formula I, Xaa at position 22 is Gly, Glu, Asp, or Lys.

In another embodiment of the GLP-1 derivative of formula I, Xaa at position 23 is Gln, Glu, Asp, or Lys.

In another embodiment of the GLP-1 derivative of formula I, Xaa at position 24 is Ala, Glu, Asp, or Lys.

In another embodiment of the GLP-1 derivative of formula I, Xaa at position 25 is Ala, Glu, Asp, or Lys.

In another embodiment of the GLP-1 derivative of formula I, Xaa at position 26 is Lys, Glu, Asp, or Arg.

In another embodiment of the GLP-1 derivative of formula I, Xaa at position 27 is Glu, Asp, or Lys.

In another embodiment of the GLP-1 derivative of formula I, Xaa at position 30 is Ala, Glu, Asp, or Lys.

In another embodiment of the GLP-1 derivative of formula I, Xaa at position 31 is Trp, Glu, Asp, or Lys.

In another embodiment of the GLP-1 derivative of formula I, Xaa at position 32 is Leu, Glu, Asp, or Lys.

In another embodiment of the GLP-1 derivative of formula I, Xaa at position 33 is Val, Glu, Asp, or Lys.

In another embodiment of the GLP-1 derivative of formula I, Xaa at position 34 is Lys, Arg, Glu, or Asp.

In another embodiment of the GLP-1 derivative of formula I, Xaa at position 35. is Gly, Glu, Asp, or Lys.

In another embodiment of the GLP-1 derivative of formula I, Xaa at position 36 is Arg, Lys, Glu, or Asp.

In another embodiment of the GLP-1 derivative of formula I, Xaa at position 37 is Gly, Glu, Asp, or Lys.

In another embodiment of the GLP-1 derivative of formula I, Xaa at position 38 is Arg, or Lys, or is deleted.

In another embodiment of the GLP-1 derivative of formula I, Xaa at position 39 is deleted.

In another embodiment of the GLP-1 derivative of formula I, Xaa at position 40 is deleted.

In another embodiment of the GLP-1 derivative of formula I, Xaa at position 41 is deleted.

In another embodiment of the GLP-1 derivative of formula I, Xaa at position 42 is deleted..

In another embodiment. of the GLP-1 derivative of formula I, Xaa at position 43 is deleted.

In another embodiment of the GLP-1 derivative of formula I, Xaa at position 44 is deleted.

In another embodiment of the GLP-1 derivative of formula I, Xaa at position 45 is deleted.

In another embodiment of the GLP-1 derivative of formula I, Xaa at position 26 is Arg, each of Xaa at positions 37-45 is deleted, and each of the other Xaa is the amino acid in native GLP-1(7-36).

In another embodiment of the GLP-1 derivative of formula I, Xaa at position 26 is Arg, each of Xaa at positions 38-45. is deleted, and each of the other Xaa is the amino acid in native GLP-1(7-37).

In another embodiment of the GLP-1 derivative of formula I, Xaa at position 26. is Arg, each of Xaa at positions 39-45 is deleted, and each of the other Xaa is the amino acid in native GLP-1(7-38).

In another embodiment of the GLP-1 derivative of formula I, Xaa at position 34. is Arg, each of Xaa at positions 37-45 is deleted, and each of the other. Xaa is the amino acid in native GLP-1(7-36).

In another embodiment of the GLP-1 derivative of formula I, Xaa at position 34 is Arg, each of Xaa at positions 38-45 is deleted, and each of the other Xaa is the amino. acid in native GLP-1(7-37).

In another embodiment of the GLP-1 derivative of formula I, Xaa at position 34 is Arg, each of Xaa at positions 39-45 is deleted, and each of the other Xaa is the amino acid in native GLP-1(7-38).

In another embodiment of the GLP-1 derivative of formula I, Xaa at positions 26 and 34 is Arg, Xaa at position 36 is Lys, each of Xaa at positions 37-45 is deleted, and each of the other Xaa is the amino acid in native GLP-1(7-36).

In another embodiment of the GLP-1 derivative of formula I, Xaa at positions 26 and 34 is Arg, Xaa at position 36 is Lys, each of Xaa at positions 38-45 is deleted, and each of the other Xaa is the amino acid in native GLP-1(7-37).

In another embodiment of the GLP-1 derivative of formula I, Xaa at positions 26 and 34 is Arg, Xaa at position 36 is Lys, each of Xaa at positions 39-45 is deleted, and each of the other Xaa is the amino acid in native GLP-1(7-38).

In another embodiment of the GLP-1 derivative of formula I, Xaa at positions 26 and 34 is Arg, Xaa at position 38 is Lys, each of Xaa at positions 39-45 is deleted, and each of the other Xaa is the amino acid in native GLP-1(7-38).

In another embodiment of the GLP-1 derivative of formula I, Xaa at position 8 is Thr, Ser, Gly or Val, Xaa at position 37 is Glu, Xaa at position 36 is Lys, each of Xaa at positions 38-45 is deleted, and each of the other Xaa is the amino acid in native GLP-1(7-37).

In another embodiment of the GLP-1 derivative of formula I, Xaa at position 8 is Thr, Ser, Gly or Val, Xaa at position 37 is Glu, Xaa at position 36 is Lys, each of Xaa at positions 39-45 is deleted, and each of the other Xaa is the amino acid in native GLP-1(7-38).

In another embodiment of the GLP-1 derivative of formula I, Xaa at position 8 is Thr, Ser, Gly or Val, Xaa at position 37 is Glu, Xaa at position 38 is Lys, each of Xaa at positions 39-45 is deleted, and each of the other Xaa is the amino acid in native GLP-1(7-38).

In another embodiment of the GLP-1 derivative of formula I, Xaa at position 18, 23 or 27 is Lys, and Xaa at positions 26 and 34 is Arg, each of Xaa at positions 37-45 is deleted, and each of the other Xaa is the amino acid in native GLP-1(7-36).

In another embodiment of the GLP-1 derivative of formula I, Xaa at position 18, 23 or 27 is Lys, and Xaa at positions 26 and 34 is Arg, each of Xaa at positions 38-45 is deleted, and each of the other Xaa is the amino acid in native GLP-1(7-37).

In another embodiment of the GLP-1 derivative of formula I, Xaa at position 18, 23 or 27 is Lys, and Xaa at positions 26 and 34 is Arg, each of Xaa at positions 39-45 is deleted, and each of the other Xaa is the amino acid in native GLP-1(7-38).

In another embodiment of the GLP-1 derivative of formula I, Xaa at position 8 is Thr, Ser, Gly, or Val, Xaa at position 18, 23 or 27. is Lys, and Xaa at position 26 and 34 is Arg, each of Xaa at positions 37-45 is deleted, and each of the other Xaa is the amino. acid in native GLP-1(7-36).

In another embodiment of the GLP-1 derivative of formula I, Xaa at position 8 is Thr, Ser, Gly, or Val, Xaa at position 18, 23 or 27 is Lys, and Xaa at position 26 and 34 is Arg, each of Xaa at positions 38-45 is deleted, and each of the other Xaa is the amino acid in native GLP-1(7-37).

In another embodiment of the GLP-1 derivative of formula I, Xaa at position 8 is Thr, Ser, Gly, or Val, Xaa at position 18, 23 or 27 is Lys, and Xaa at position 26 and 34 is Arg, each of Xaa at positions 39-45 is deleted, and each of the other Xaa is the amino acid in native GLP-1(7-38).

Such GLP-1 derivatives includes, but is not limited to,
Lys³⁴ (N^{ε}-(γ-glutamyl(N^{α}-tetradecanoyl))) GLP-1 (7-37),
Arg^{26,34},Lys⁸(N^{ε}-(γ-glutamyl(N^{α}-hexadecanoyl))) GLP-1 (7-37),
Arg³⁴,Lys²⁶(N^{ε}-(γ-glutamyl(N^{α}-dodecanoyl))) GLP-1(7-37),
Arg³⁴,Lys²⁶(N^{ε}-(β-alanyl(N^{α}-hexadecanoyl))) GLP-1 (7-37),
Arg³⁴,Lys²⁶(N^{ε}-(α-glutamyl(N^{α}-hexadecanoyl))) GLP-1 (7-37),
Arg³⁴,Lys²⁶(N^{ε}-(piperidinyl-4-carbonyl(N-hexadecanoyl))) GLP-1 (7-37),
Arg³⁴,Lys²⁶(N^{ε}-(γ-glutamyl(N^{α}-decanoyl))) GLP-1 (7-37),
(NNC 90-1182).Glu^{22,23,30}Arg^{26,34}Lys³⁸(N^{ε}-(γ-glutamyl(N^{α}-tetradecanoyl)))-GLP-1(7-38)-OH, (NNC 90-1158).
Glu^{23,26}Arg³⁴Lys³⁸(N^{ε}-(γ-glutamyl(N^{α}-tetradecanoyl)))-GLP-1(7-38)-OH,
Lys^{26,34}-bis(N^{ε}-(γ-glutamyl(N^{α}-tetradecanoyl)))-GLP-1(7-37)-OH,
Lys^{26,34}-bis(N^{ε}-(γ-glutamyl(N^{α}-hexadecanoyl)))-GLP-1(7-37)-OH, (NNC 90-1169).
Arg³⁴Lys²⁶(N^{ε}-(γ-glutamyl(N^{α}-hexadecanoyl)))-GLP-1(7-37)-OH, (NNC 90-1170).
Arg^{26,34}Lys³⁸(N^{ε}-(γ-glutamyl(N^{α}-tetradecanoyl)))-GLP-1(7-38)-OH, (NNC 90-1171).
Arg^{26,34}Lys³⁸(N^{ε}-(γ-glutamyl(N^{α}-hexadecanoyl)))-GLP-1(7-38)-OH, (NNC 90-1173).
Arg³⁴Lys²⁶(N^{ε}-(γ-glutamyl(N^{α}-tetradecanoyl)))-GLP-1(7-37)-OH, (NNC 90-1179).
Arg^{26,34}Lys³⁸(N^{ε}-(γ-glutamyl(N^{α}-octadecanoyl)))-GLP-1(7-38)-OH, (NNC 90-1182).
Glu^{22,23,30}Arg^{26,34}Lys³⁸(N^{ε}-(γ-glutamyl(N^{α}-tetradecanoyl)))-GLP-1(7-38)-OH, (NNC 90-1158).
Glu^{23,26}Arg³⁴Lys³⁸(N^{ε}-(γ-glutamyl(N^{α}-tetradecanoyl)))-GLP-1(7-38)-OH,
Lys^{26,34}-bis(N^{ε}-(ω-carboxytridecanoyl))-GLP-1(7-37)-OH,
Lys^{26,34}-bis(N^{ε}-(γ-glutamyl(N^{α}-tetradecanoyl)))-GLP-1(7-37)-OH,
Arg^{26,34}Lys³⁸(N^{ε}-(ω-carboxypentadecanoyl))-GLP-1(7-38)-OH, (NNC 90-1168).
Lys^{26,34}-bis(N^{ε}-(γ-glutamyl(N^{α}-hexadecanoyl)))-GLP-1(7-37)-OH, (NNC 90-1169).
Arg³⁴Lys²⁶(N^{ε}-(γ-glutamyl(N^{α}-hexadecanoyl)))-GLP-1(7-37)-OH, (NNC 90-1170).
Arg^{26,34}Lys³⁸(N^{ε}-(γ-glutamyl(N^{α}-tetradecanoyl)))-GLP-1(7-38)-OH, (NNC 90-1171).
Arg^{26,34}Lys³⁸(N^{ε}-(ω-carboxypentadecanoyl))-GLP-1(7-38)-OH, (NNC 90-1172).
Arg^{26,34}Lys³⁸(N^{ε}-(γ-glutamyl(N^{α}-hexadecanoyl)))-GLP-1(7-38)-OH, (NNC 90-1173).
Arg^{18,23,26,30,34}Lys³⁸(N^{ε}-hexadecanoyl)-GLP-1(7-38)-OH,
Arg^{26,34}Lys³⁸(N^{ε}-(ω-carboxytridecanoyl))-GLP-1(7-38)-OH, (NNC 90-1177)
Arg³⁴Lys²⁶(N^{ε}-(γ-glutamyl(N^{α}-tetradecanoyl)))-GLP-1(7-37)-OH, (NNC 90-1179).
Arg^{26,34}Lys³⁸(N^{ε}-(γ-glutamyl(N^{α}-octadecanoyl)))-GLP-1(7-38)-OH, (NNC 90-1182).
(NNC 90-1182).Glu^{22,23,30}Arg^{26,34}Lys³⁸(N^{ε}-(β-alanyl(N^{α}-tetradecanoyl)))-GLP-1(7-38)-OH, (NNC 90-1158).
Glu^{23,26}Arg³⁴Lys³⁸(N^{ε}-(β-alanyl(N^{α}-tetradecanoyl)))-GLP-1(7-38)-OH,
Lys^{26,34}-bis(N^{ε}-(β-alanyl(N^{α}-tetradecanoyl)))-GLP-1(7-37)-OH,
Lys^{26,34}-bis(N^{ε}-(β-alanyl(N^{α}-hexadecanoyl)))-GLP-1(7-37)-OH, (NNC 90-1169).
Arg³⁴Lys²⁶(N^{ε}-(β-alanyl(N^{α}-hexadecanoyl)))-GLP-1(7-37)-OH, (NNC 90-1170).
Arg^{26,34}Lys³⁸(N^{ε}-(β-alanyl(N^{α}-tetradecanoyl)))-GLP-1(7-38)-OH, (NNC 90-1171).
Arg^{26,34}Lys³⁸(N^{ε}-(β-alanyl(N^{α}-hexadecanoyl)))-GLP-1(7-38)-OH, (NNC 90-1173).
Arg³⁴Lys²⁶(N^{ε}-(β-alanyl(N^{α}-tetradecanoyl)))-GLP-1(7-37)-OH, (NNC 90-1179).
Arg^{26,34}Lys³⁸(N^{ε}-(β-alanyl(N^{α}-octadecanoyl)))-GLP-1(7-38)-OH, (NNC 90-1182).
Glu^{22,23,30}Arg^{26,34}Lys³⁸(N^{ε}-(β-alanyl(N^{α}-tetradecanoyl)))-GLP-1(7-38)-OH, (NNC 90-1158).
Glu^{23,26}Arg³⁴Lys³⁸(N^{ε}-(β-alanyl(N^{α}-tetradecanoyl)))-GLP-1(7-38)-OH,
Lys^{26,34}-bis(N^{ε}-(β-alanyl(N^{α}-tetradecanoyl)))-GLP-1(7-37)-OH,
Lys^{26,34}-bis(N^{ε}-(β-alanyl(N^{α}-hexadecanoyl)))-GLP-1(7-37)-OH, (NNC 90-1169).
Arg³⁴Lys²⁶(N^{ε}-(β-alanyl(N^{α}-hexadecanoyl)))-GLP-1(7-37)-OH, (NNC 90-1170).
Arg^{26,34}Lys³⁸(N^{ε}-(β-alanyl(N^{α}-tetradecanoyl)))-GLP-1(7-38)-OH, (NNC 90-1171).
Arg^{26,34}Lys³⁸(N^{ε}-(β-alanyl(N^{α}-hexadecanoyl)))-GLP-1(7-38)-OH, (NNC 90-1173).
Arg³⁴Lys²⁶(N^{ε}-(β-alanyl(N^{α}-tetradecanoyl)))-GLP-1(7-37)-OH, (NNC 90-1179).
Arg^{26,34}Lys³⁸(N^{ε}-(β-alanyl(N^{α}-octadecanoyl)))-GLP-1(7-38)-OH, (NNC 90-1182).
Lys²⁶(N^{ε}-tetradecanoyl)-GLP-1(7-37);
Lys³⁴(N^{ε}-tetradecanoyl)-GLP-1(7-37);
Lys^{26,34}-bis(N^{ε}-tetradecanoyl)-GLP-1(7-37);
Gly⁸Lys²⁶(N^{ε}-tetradecanoyl)-GLP-1(7-37);
Gly⁸Lys³⁴(N^{ε}-tetradecanoyl)-GLP-1(7-37);
Gly⁸Lys^{26,34}-bis(N^{ε}-tetradecanoyl)-GLP-1(7-37);
Val⁸Lys²⁶(N^{ε}-tetradecanoyl)-GLP-1(7-37);
Val⁸Lys³⁴(N^{ε}-tetradecanoyl)-GLP-1(7-37);
Val⁸Lys^{26,34}-bis(N^{ε}-tetradecanoyl)-GLP-1(7-37);
Arg²⁶Lys³⁴(N^{ε}-tetradecanoyl)-GLP-1(7-37);
Lys²⁶(N^{ε}-tetradecanoyl)-GLP-1(7-38);
Lys³⁴(N^{ε}-tetradecanoyl)-GLP-1(7-38);
Lys^{26,34}-bis(N^{ε}-tetradecanoyl)-GLP-1(7-38);
Gly⁸Lys²⁶(N^{ε}-tetradecanoyl)-GLP-1(7-38);
Gly⁸Lys³⁴(N^{ε}-tetradecanoyl)-GLP-1(7-38);
Gly⁸Lys^{26,34}-bis(N^{ε}-tetradecanoyl)-GLP-1(7-38);
Arg²⁶Lys³⁴(N^{ε}-tetradecanoyl)-GLP-1(7-38);
Lys²⁶(N^{ε}-tetradecanoyl)-GLP-1(7-39);
Lys³⁴(N^{ε}-tetradecanoyl)-GLP-1(7-39);
Lys^{26,34}-bis(N^{ε}-tetradecanoyl)-GLP-1(7-39);
Gly⁸Lys²⁶(N^{ε}-tetradecanoyl)-GLP-1(7-39);
Gly⁸Lys³⁴(N^{ε}-tetradecanoyl)-GLP-1(7-39);
Gly⁸Lys^{26,34}-bis(N^{ε}-tetradecanoyl)-GLP-1(7-39);
Arg²⁶Lys³⁴(N^{ε}-tetradecanoyl)-GLP-1(7-39);
Lys²⁶(N^{ε}-tetradecanoyl)-GLP-1(7-40);
Lys³⁴(N^{ε}-tetradecanoyl)-GLP-1(7-40);
Lys^{26,34}-bis(N^{ε}-tetradecanoyl)-GLP-1(7-40);
Gly⁸Lys²⁶(N^{ε}-tetradecanoyl)-GLP-1(7-40);
Gly⁸Lys³⁴(N^{ε}-tetradecanoyl)-GLP-1(7-40);
Gly⁸Lys^{26,34}-bis(N^{ε}-tetradecanoyl)-GLP-1(7-40);
Arg²⁶Lys³⁴(N^{ε}-tetradecanoyl)-GLP-1(7-40);
Lys²⁶(N^{ε}-tetradecanoyl)-GLP-1(7-36);
Lys³⁴(N^{ε}-tetradecanoyl)-GLP-1(7-36);
Lys^{26,34}-bis(N^{ε}-tetradecanoyl)-GLP-1(7-36);
Gly⁸Lys²⁶(N^{ε}-tetradecanoyl)-GLP-1(7-36);
Gly⁸Lys³⁴(N^{ε}-tetradecanoyl)-GLP-1(7-36);
Gly⁸Lys^{26,34}-bis(N^{ε}-tetradecanoyl)-GLP-1(7-36);
Arg²⁶Lys³⁴(N^{ε}-tetradecanoyl)-GLP-1(7-36);
Lys²⁶(N^{ε}-tetradecanoyl)-GLP-1(7-35);
Lys³⁴(N^{ε}-tetradecanoyl)-GLP-1(7-35);
Lys^{26,34}-bis(N^{ε}-tetradecanoyl)-GLP-1(7-35);
Gly⁸Lys²⁶(N^{ε}-tetadecanoyl)-GLP-1(7-35);
Gly⁸Lys³⁴(N^{ε}-tetradecanoyl)-GLP-1(7-35);
Gly⁸Lys^{26,34}-bis(N^{ε}-tetradecanoyl)-GLP-1(7-35);
Arg²⁶Lys³⁴(N^{ε}-tetradecanoyl)-GLP-1(7-35);
Lys²⁶(N^{ε}-tetradecanoyl)-GLP-1(7-36)amide;
Lys³⁴(N^{ε}-tetradecanoyl)-GLP-1(7-36)amide;
Lys^{26,34}-bis(N^{ε}-tetradecanoyl)-GLP-1(7-36)amide;
Gly⁸Lys²⁶(N^{ε}-tetradecanoyl)-GLP-1(7-36)amide;
Gly⁸Lys³⁴(N^{ε}-tetradecanoyl)-GLP-1(7-36)amide;
Gly⁸Lys^{26,34}-bis(N^{ε}-tetradecanoyl)-GLP-1(7-36)amide;
Arg²⁶Lys³⁴(N^{ε}-tetradecanoyl)-GLP-1(7-36)amide;
Gly⁸Arg²⁶Lys³⁴(N^{ε}-tetradecanoyl)-GLP-1(7-37);
Lys²⁶(N^{ε}-tetradecanoyl)Arg³⁴-GLP-1(7-37);
Gly⁸Lys²⁶(N^{ε}-tetradecanoyl)Arg³⁴-GLP-1(7-37);
Arg^{26,34}Lys³⁶(N^{ε}-tetradecanoyl)-GLP-1(7-37);
Gly⁸Arg^{26,34}Lys³⁶(N^{ε}-tetradecanoyl)-GLP-1(7-37);
Gly⁸Arg²⁶Lys³⁴(N^{ε}-tetradecanoyl)-GLP-1(7-38);
Lys²⁶(N^{ε}-tetradecanoyl)Arg³⁴-GLP-1(7-38);
Gly⁸Lys²⁶(N^{ε}-tetradecanoyl)Arg³⁴-GLP-1(7-38);
Arg^{26,34}Lys³⁶(N^{ε}-tetradecanoyl)-GLP-1(7-38);
Arg^{26,34}Lys³⁸(N^{ε}-tetradecanoyl)-GLP-1(7-38);
Gly⁸Arg^{26,34}Lys³⁶(N^{ε}-tetradecanoyl)-GLP-1(7-38);
Gly⁸Arg²⁶Lys³⁴(N^{ε}-tetradecanoyl)-GLP-1(7-39);
Lys²⁶(N^{ε}-tetradecanoyl)Arg³⁴-GLP-1(7-39);
Gly⁸Lys²⁶(N^{ε}-tetradecanoyl)Arg³⁴-GLP-1(7-39);
Arg^{26,34}Lys³⁶(N^{ε}-tetradecanoyl)-GLP-1(7-39);
Gly⁸Arg^{26,34}Lys³⁶(N^{ε}-tetradecanoyl)-GLP-1(7-39);
Gly⁸Arg²⁶Lys³⁴(N^{ε}-tetradecanoyl)-GLP-1(7-40);
Lys²⁶(N^{ε}-tetradecanoyl)Arg³⁴-GLP-1(7-40);
Gly⁸Lys²⁶(N^{ε}-tetradecanoyl)Arg³⁴-GLP-1(7-40);
Arg^{26,34}Lys³⁶(N^{ε}-tetradecanoyl)-GLP-1(7-40);
Gly⁸Arg^{26,34}Lys³⁶(N^{ε}-tetradecanoyl)-GLP-1(7-40);
Lys²⁶(N^{ε}-(ω-carboxynonadecanoyl)-GLP-1(7-37);
Lys³⁴(N^{ε}-(ω-carboxynonadecanoyl))-GLP-1(7-37);
Lys^{26,34}-bis(N^{ε}-(ω-carboxynonadecanoyl))-GLP-1(7-37);
Gly⁸Lys²⁶(N^{ε}-(ω-carboxynonadecanoyl))-GLP-1(7-37);
Gly⁸Lys³⁴(N^{ε}-(ω-carboxynonadecanoyl))-GLP-1(7-37);
Gly⁸Lys^{26,34}-bis(N^{ε}-(ω-carboxynonadecanoyl))-GLP-1(7-37);
Lys²⁶(N^{ε}-(ω-carboxynonadecanoyl))-GLP-1(7-38);
Lys³⁴(N^{ε}-(ω-carboxynonadecanoyl))-GLP-1(7-38);
Lys^{26,34}-bis(N^{ε}-(ω-carboxynonadecanoyl))-GLP-1(7-38);
Gly⁸Lys²⁶(N^{ε}-(ω-carboxynonadecanoyl))-GLP-1(7-38);
Gly⁸Lys³⁴(N^{ε}-(ω-carboxynonadecanoyl))-GLP-1(7-38);
Gly⁸Lys^{26,34}-bis(N^{ε}-(ω-carboxynonadecanoyl))-GLP-1(7-38);
Lys²⁶(N^{ε}-(ω-carboxynonadecanoyl))-GLP-1(7-39);
Lys³⁴(N^{ε}-(ω-carboxynonadecanoyl))-GLP-1(7-39);
Lys^{26,34}-bis(N^{ε}-(ω-carboxynonadecanoyl))-GLP-1(7-39);
Gly⁸Lys²⁶(N^{ε}-(ω-carboxynonadecanoyl))-GLP-1(7-39);
Gly⁸Lys³⁴(N^{ε}-(ω-carboxynonadecanoyl))-GLP-1(7-39);
Gly⁸Lys^{26,34}-bis(N^{ε}-(ω-carboxynonadecanoyl))-GLP-1(7-39);
Lys²⁶(N^{ε}-(ω-carboxynonadecanoyl))-GLP-1(7-40);
Lys³⁴(N^{ε}-(ω-carboxynonadecanoyl))-GLP-1(7-40);
Lys^{26,34}-bis(N^{ε}-(ω-carboxynonadecanoyl))-GLP-1(7-40);
Gly⁸Lys²⁶(N^{ε}-(ω-carboxynonadecanoyl))-GLP-1(7-40);
Gly⁸Lys³⁴(N^{ε}-(ω-carboxynonadecanoyl))-GLP-1(7-40);
Gly⁸Lys^{26,34}-bis(N^{ε}-(ω-carboxynonadecanoyl))-GLP-1(7-40);
Lys²⁶(N^{ε}-(ω-carboxynonadecanoyl))-GLP-1(7-36);
Lys³⁴(N^{ε}-(ω-carboxynonadecanoyl))-GLP-1(7-36);
Lys^{26,34}-bis(N^{ε}-(ω-carboxynonadecanoyl))-GLP-1(7-36);
Gly⁸Lys²⁶(N^{ε}-(ω-carboxynonadecanoyl))-GLP-1(7-36);
Gly⁸Lys³⁴(N^{ε}-(ω-carboxynonadecanoyl))-GLP-1(7-36);
Gly⁸Lys^{26,34}-bis(N^{ε}-(ω-carboxynonadecanoyl))-GLP-1(7-36);
Lys²⁶(N^{ε}-(ω-carboxynonadecanoyy))-GLP-1(7-36)amide;
Lys³⁴(N^{ε}-(ω-carboxynonadecanoyl))-GLP-1(7-36)amide;
Lys^{26,34}-bis(N^{ε}-(ω-carboxynonadecanoyl))-GLP-1(7-36)amide;
Gly⁸Lys²⁶(N^{ε}-(ω-carboxynonadecanoyl))-GLP-1(7-36)amide;
Gly⁸Lys³⁴(N^{ε}-(ω-carboxynonadecanoyl))-GLP-1(7-36)amide;
Gly⁸Lys^{26,34}-bis(N^{ε}-(ω-carboxynonadecanoyl))-GLP-1(7-36)amide;
Lys²⁶(N^{ε}-(ω-carboxynonadecanoyl))-GLP-1(7-35);
Lys³⁴(N^{ε}-(ω-carboxynonadecanoyl))-GLP-1(7-35);
Lys^{26,34}-bis(N^{ε}-(ω-carboxynonadecanoyl))-GLP-1(7-35);
Gly⁸Lys²⁶(N^{ε}-(ω-carboxynonadecanoyl))-GLP-1(7-35);
Gly⁸Lys³⁴(N^{ε}-(ω-carboxynonadecanoyl))-GLP-1(7-35);
Gly⁸Lys^{26,34}-bis(N^{ε}-(ω-carboxynonadecanoyl))-GLP-1(7-35);
Arg²⁶Lys³⁴(N^{ε}-(ω-carboxynonadecanoyl))-GLP-1(7-37);
Gly⁸Arg²⁶Lys³⁴(N^{ε}-(ω-carboxynonadecanoyl))-GLP-1(7-37);
Lys²⁶(N^{ε}-(ω-carboxynonadecanoyl))Arg³⁴-GLP-1(7-37);
Gly⁸Lys²⁶(N^{ε}-(ω-carboxynonadecanoyl))Arg³⁴-GLP-1(7-37);
Arg^{26,34}Lys³⁶(N^{ε}-(ω-carboxynonadecanoyl))-GLP-1(7-37);
Gly⁸Arg^{26,34}Lys³⁶(N^{ε}-(ω-carboxynonadecanoyl))-GLP-1(7-37);
Arg²⁶Lys³⁴(N^{ε}-(ω-carboxynonadecanoyl))-GLP-1(7-38);
Gly⁸Arg²⁶Lys³⁴(N^{ε}-(ω-carboxynonadecanoyl))-GLP-1(7-38);
Lys²⁶(N^{ε}-(ω-carboxynonadecanoyl))Arg³⁴-GLP-1(7-38);
Gly⁸Lys²⁶(N^{ε}-(ω-carboxynonadecanoyl))Arg³⁴-GLP-1(7-38);
Arg^{26,34}Lys³⁶(N^{ε}-(ω-carboxynonadecanoyl))-GLP-1(7-38);
Arg^{26,34}Lys³⁸(N^{ε}-(ω-carboxynonadecanoyl))-GLP-1(7-38);
Gly⁸Arg^{26,34}Lys³⁶(N^{ε}-(ω-carboxynonadecanoyl))-GLP-1(7-38);
Arg²⁶Lys³⁴(N^{ε}-(ω-carboxynonadecanoyl))-GLP-1(7-39);
Gly⁸Arg²⁶Lys³⁴(N^{ε}-(ω-carboxynonadecanoyl))-GLP-1(7-39);
Gly⁸Lys²⁶(N^{ε}-(ω-carboxynonadecanoyl))Arg³⁴-GLP-1(7-39);
Gly⁸Lys²⁶(N^{ε}-(ω-carboxynonadecanoyl))Arg³⁴-GLP-1(7-39);
Gly⁸Arg^{26,34}Lys³⁶(N^{ε}-(ω-carboxynonadecanoyl))-GLP-1(7-39);
Gly⁸Arg^{26,34}Lys³⁶(N^{ε}-(ω-carboxynonadecanoyl))-GLP-1(7-39);
Gly⁸Arg²⁶Lys³⁴(N^{ε}-(ω-carboxynonadecanoyl))-GLP-1(7-40);
Gly⁸Arg²⁶Lys³⁴(N^{ε}-(ω-carboxynonadecanoyl))-GLP-1(7-40);
Lys²⁶(N^{ε}-(ω-carboxynonadecanoyl))Arg³⁴-GLP-1(7-40);
Gly⁸Lys²⁶(N^{ε}-(ω-carboxynonadecanoyl))Arg³⁴-GLP-1(7-40);
Arg^{26,34}Lys³⁶(N^{ε}-(ω-carboxynonadecanoyl))-GLP-1(7-40);
Gly⁸Arg^{26,34}Lys³⁶(N^{ε}-(ω-carboxynonadecanoyl))-GLP-1(7-40);
Lys²⁶(N^{ε}-(7-deoxycholoyl))-GLP-1(7-37);
Lys³⁴(N^{ε}-(7-deoxycholoyl))-GLP-1(7-37);
Lys^{26,34}-bis(N^{ε}-(7-deoxycholoyl))-GLP-1(7-37);
Gly⁸Lys²⁶(N^{ε}-(7-deoxycholoyl))-GLP-1(7-37);
Gly⁸Lys³⁴(N^{ε}-(7-deoxycholoyl))-GLP-1(7-37);
Gly⁸Lys^{26,34}-bis(N^{ε}-(7-deoxycholoyl))-GLP-1(7-37);
Arg²⁶Lys³⁴(N^{ε}-(7-deoxycholoyl))-GLP-1(7-37);
Lys²⁶(N^{ε}-(7-deoxycholoyl))-GLP-1(7-38);
Lys³⁴(N^{ε}-(7-deoxycholoyl))-GLP-1(7-38);
Lys^{26,34}-bis(N^{ε}-(7-deoxycholoyl))-GLP-1(7-38);
Gly⁸Lys²⁶(N^{ε}-(7-deoxycholoyl))-GLP-1(7-38);
Gly⁸Lys³⁴(N^{ε}-(7-deoxycholoyl))-GLP-1(7-38);
Gly⁸Lys^{26,34}-bis(N^{ε}-(7-deoxycholoyl))-GLP-1(7-38);
Arg²⁶Lys³⁴(N^{ε}-(7-deoxycholoyl))-GLP-1(7-38);
Lys²⁶(N^{ε}-(7-deoxycholoyl))-GLP-1(7-39);
Lys³⁴(N^{ε}-(7-deoxycholoyl))-GLP-1(7-39);
Lys^{26,34}-bis(N^{ε}-(7-deoxycholoyl))-GLP-1(7-39);
Gly⁸Lys²⁶(N^{ε}-(7-deoxycholoyl))-GLP-1(7-39);
Gly⁸Lys³⁴(N^{ε}-(7-deoxycholoyl))-GLP-1(7-39);
Gly⁸Lys^{26,34}-bis(N^{ε}-(7-deoxycholoyl))-GLP-1(7-39); Arg²⁶Lys³⁴(N^{ε}-(7-deoxycholoyl)-GLP-1(7-39);
Lys²⁶(N^{ε}-(7-deoxycholoyl))-GLP-1(7-40);
Lys³⁴(N^{ε}-(7-deoxycholoyl))-GLP-1(7-40);
Lys^{26,34}-bis(N^{ε}-(7-deoxycholoyl))-GLY-1(7-40);
Gly⁸Lys²⁶(N^{ε}-(7-deoxycholoyl))-GLP-1(7-40);
Gly⁸Lys³⁴(N^{ε}-(7-deoxycholoyl))-GLP-1(7-40);
Gly⁸Lys^{26,34}-bis(N^{ε}-(7-deoxycholoyl))-GLP-1(7-40);
Arg²⁶Lys³⁴(N^{ε}-(7-deoxycholoyl))-GLP-1(7-40);
Lys²⁶(N^{ε}-(7-deoxycholoyl))-GLP-1(7-36);
Lys³⁴(N^{ε}-(7-deoxycholoyl))-GLP-1(7-36);
Lys^{26,34}-bis(N^{ε}-(7-deoxycholoyl))-GLP-1(7-36);
Gly⁸Lys²⁶(N^{ε}-(7-deoxycholoyl))-GLP-1(7-36);
Gly⁸Lys³⁴(N^{ε}-(7-deoxycholoyl))-GLP-1(7-36);
Gly⁸Lys^{26,34}-bis(N^{ε}-(7-deoxycholoyl))-GLP-1(7-36);
Arg²⁶Lys³⁴(N^{ε}-(7-deoxycholoyl))-GLP-1(7-36);
Lys²⁶(N^{ε}-(7-deoxycholoyl))-GLP-1(7-35);
Lys³⁴(N^{ε}-(7-deoxycholoyl))-GLP-1(7-35);
Lys^{26,34}-bis(N^{ε}-(7-deoxycholoyl))-GLP-1(7-35);
Gly⁸Lys²⁶(N^{ε}-(7-deoxycholoyl))-GLP-1(7-35);
Gly⁸Lys³⁴(N^{ε}-(7-deoxycholoyl))-GLP-1(7-35);
Gly⁸Lys^{26,34}-bis(N^{ε}-(7-deoxycholoyl))-GLP-1(7-35);
Arg²⁶Lys³⁴(N^{ε}-(7-deoxycholoyl))-GLP-1(7-35);
Lys²⁶(N^{ε}-(7-deoxycholoyl))-GLP-1(7-36)amide;
Lys³⁴(N^{ε}-(7-deoxycholoyl))-GLP-1(7-36)amide;
Lys^{26,34}-bis(N^{ε}-(7-deoxycholoyl))-GLP-1(7-36)amide;
Gly⁸Lys²⁶(N^{ε}-(7-deoxycholoyl))-GLP-1(7-36)amide;
Gly⁸Lys³⁴(N^{ε}-(7-deoxycholoyl))-GLP-1(7-36)amide;
Gly⁸Lys^{26,34}-bis(N^{ε}-(7-deoxycholoyl))-GLP-1(7-36)amide;
Arg²⁶Lys³⁴(N^{ε}-(7-deoxycholoyl))-GLP-1(7-36)amide;
Gly⁸Arg²⁶Lys³⁴(N^{ε}-(7-deoxycholoyl))-GLP-1(7-37);
Lys²⁶(N^{ε}-(7-deoxycholoyl))Arg³⁴-GLP-1(7-37);
Gly⁸Lys²⁶(N^{ε}-(7-deoxycholoyl))Arg³⁴-GLP-1(7-37);
Arg^{26,34}Lys³⁶(N^{ε}-(7-deoxycholoyl))-GLP-1(7-37);
Gly⁸Arg^{26,34}Lys³⁶(N^{ε}-(7-deoxycholoyl))-GLP-1(7-37);
Lys²⁶(N^{ε}-(choloyl))-GLP-1(7-37);
Lys³⁴(N^{ε}-(choloyl))-GLP-1(7-37);
Lys^{26,34}-bis(N^{ε}-(choloyl))-GLP-1(7-37);
Gly⁸Lys²⁶(N^{ε}-(choloyl))-GLP-1(7-37);
Gly⁸Lys³⁴(N^{ε}-(choloyl))-GLP-1(7-37);
Gly⁸Lys^{26,34}-bis(N^{ε}-(choloyl))-GLP-1(7-37);
Arg²⁶Lys³⁴(N^{ε}-(choloyl))-GLP-1(7-37);
Gly⁸Arg²⁶Lys³⁴(N^{ε}-(7-deoxycholoyl))-GLP-1(7-38);
Lys²⁶(N^{ε}-(7-deoxycholoyl))Arg³⁴-GLP-1(7-38);
Gly⁸Lys²⁶(N^{ε}-(7-deoxycholoyl))Arg³⁴-GLP-1(7-38);
Arg^{26,34}Lys³⁶(N^{ε}-(7-deoxycholoyl))-GLP-1(7-38);
Arg^{26,34}Lys³⁸(N^{ε}-(7-deoxycholoyl))-GLP-1(7-38);
Gly⁸Arg^{26,34}Lys³⁶(N^{ε}-(7-deoxycholoyl))-GLP-1(7-38);
Lys²⁶(N^{ε}-(choloyl))-GLP-1(7-38);
Lys³⁴(N^{ε}-(choloyl))-GLP-1(7-38);
Lys^{26,34}-bis(N^{ε}-(choloyl))-GLP-1(7-38);
Gly⁸Lys²⁶(N^{ε}-(choloyl))-GLP-1(7-38);
Gly⁸Lys³⁴(N^{ε}-(choloyl))-GLP-1(7-38);
Gly⁸Lys^{26,34}-bis(N^{ε}-(choloyl))-GLP-1(7-38);
Arg²⁶Lys³⁴(N^{ε}-(choloyl))-GLP-1(7-38);
Gly⁸Arg²⁶Lys³⁴(N^{ε}-(7-deoxycholoyl))-GLP-1(7-39);
Lys²⁶(N^{ε}-(7-deoxycholoyl))Arg³⁴-GLP-1(7-39);
Gly⁸Lys²⁶(N^{ε}-deoxycholoyl))Arg³⁴-GLP-1(7-39);
Arg^{26,34}Lys³⁶(N^{ε}-(7-deoxycholoyl))-GLP-1(7-39);
Gly⁸Arg^{26,34}Lys³⁶(N^{ε}-(7-deoxycholoyl))-GLP-1(7-39);
Lys²⁶(N^{ε}-(choloyl))-GLP-1(7-39);
Lys³⁴(N^{ε}-(choloyl))-GLP-1(7-39);
Lys^{26,34}-bis(N^{ε}-(choloyl))-GLP-1(7-39);
Gly⁸Lys²⁶(N^{ε}-(choloyl))-GLP-1(7-39);
Gly⁸Lys³⁴(N^{ε}-(choloyl))-GLP-1(7-39);
Gly⁸Lys^{26,34}-bis(N^{ε}-(choloyl))-GLP-1(7-39);
Arg²⁶Lys³⁴(N^{ε}-(choloyl))-GLP-1(7-39);
Gly⁸Arg²⁶Lys³⁴(N^{ε}-(7-deoxycholoyl))-GLP-1(7-40);
Lys²⁶(N^{ε}-(7-deoxycholoyl))Arg³⁴-GLP-1(7-40);
Gly⁸Lys²⁶(N^{ε}-(7-deoxycholoyl))Arg³⁴-GLP-1(7-40);
Arg^{26,34}Lys³⁶(N^{ε}-(7-deoxycholoyl))-GLP-1(7-40);
Gly⁸Arg^{26,34}Lys³⁶(N^{ε}-(7-deoxycholoyl))-GLP-1(7-40);
Lys²⁶(N^{ε}-(choloyl))-GLP-1(7-40);
Lys³⁴(N^{ε}-(choloyl))-GLP-1(7-40);
Lys^{26,34}-bis(N^{ε}-(choloyl))-GLP-1(7-40);
Gly⁸Lys²⁶(N^{ε}-(choloyl))-GLP-1(7-40);
Gly⁸Lys³⁴(N^{ε}-(choloyl))-GLP-1(7-40);
Gly⁸Lys^{26,34}-bis(N^{ε}-(choloyl))-GLP-1(7-40);
Arg²⁶Lys³⁴(N^{ε}-(choloyl))-GLP-1(7-40);
Lys²⁶(N^{ε}-(choloyl))-GLP-1(7-36);
Lys³⁴(N^{ε}-(choloyl))-GLP-1(7-36);
Lys^{26,34}-bis(N^{ε}-(choloyl))-GLP-1(7-36);
Gly⁸Lys²⁶(N^{ε}-(choloyl))-GLP-1(7-36);
Gly⁸Lys³⁴(N^{ε}-(choloyl))-GLP-1(7-36);
Gly⁸Lys^{26,34}-bis(N^{ε}-(choloyl))-GLP-1(7-36);
Arg²⁶Lys³⁴(N^{ε}-(choloyl))-GLP-1(7-36);
Lys²⁶(N^{ε}-(choloyl))-GLP-1(7-35);
Lys³⁴(N^{ε}-(choloyl))-GLP-1(7-35);
Lys^{26,34}-bis(N^{ε}-(choloyl))-GLP-1(7-35);
Gly⁸Lys²⁶(N^{ε}-(choloyl))-GLP-1(7-35);
Gly⁸Lys³⁴(N^{ε}-(choloyl))-GLP-1(7-35);
Gly⁸Lys^{26,34}-bis(N^{ε}-(choloyl))-GLP-1(7-35);
Arg²⁶Lys³⁴(N^{ε}-(choloyl))-GLP-1(7-35);
Lys²⁶(N^{ε}-(choloyl))-GLP-1(7-36)amide;
Lys³⁴(N^{ε}-(choloyl))-GLP-1(7-36)amide;
Lys^{26,34}-bis(N^{ε}-(choloyl))-GLP-1(7-36)amide;
Gly⁸Lys²⁶(N^{ε}-(choloyl))-GLP-1(7-36)amide;
Gly⁸Lys³⁴(N^{ε}-(choloyl))-GLP-1(7-36)amide;
Gly⁸Lys^{26,34}-bis(N^{ε}-(choloyl))-GLP-1(7-36)amide;
Arg²⁶Lys³⁴(N^{ε}-(choloyl))-GLP-1(7-36)amide;
Gly⁸Arg²⁶Lys³⁴(N^{ε}-(choloyl))-GLP-1(7-37);
Lys²⁶(N^{ε}-(choloyl))Arg³⁴-GLP-1(7-37);
Gly⁸Lys²⁶(N^{ε}-(choloyl))Arg³⁴-GLP-1(7-37);
Arg^{26,34}Lys³⁶(N^{ε}-(choloyl))-GLP-1(7-37);
Gly⁸Arg^{26,34}Lys³⁶(N^{ε}-(choloyl))-GLP-1(7-37);
Lys²⁶(N^{ε}-(lithocholoyl))-GLP-1(7-37);
Lys³⁴(N^{ε}-(lithocholoyl))-GLP-1(7-37);
Lys^{26,34}-bis(N^{ε}-(lithocholoyl))-GLP-1(7-37);
Gly⁸Lys²⁶(N^{ε}-(lithocholoyl))-GLP-1(7-37);
Gly⁸Lys³⁴(N^{ε}-(lithocholoyl))-GLP-1(7-37);
Gly⁸Lys^{26,34}-bis(N^{ε}-(lithocholoyl))-GLP-1(7-37);
Arg²⁶Lys³⁴(N^{ε}-(lithocholoyl))-GLP-1(7-37);
Gly⁸Arg²⁶Lys³⁴(N^{ε}-(choloyl))-GLP-1(7-38);
Lys²⁶(N^{ε}-(choloyl))Arg³⁴-GLP-1(7-38);
Gly⁸Lys²⁶(N^{ε}-(choloyl))Arg³⁴-GLP-1(7-38);
Arg^{26,34}Lys³⁶(N^{ε}-(choloyl))-GLP-1(7-38);
Arg^{26,34}Lys³⁸(N^{ε}-(choloyl))-GLP-1(7-38);
Gly⁸Arg^{26,34}Lys³⁶(N^{ε}-(choloyl))-GLP-1(7-38);
Lys²⁶(N^{ε}-(lithocholoyl))-GLP-1(7-38);
Lys³⁴(N^{ε}-(lithocholoyl))-GLP-1(7-38);
Lys^{26,34}-bis(N^{ε}-(lithocholoyl))-GLP-1(7-38);
Gly⁸Lys²⁶(N^{ε}-(lithocholoyl))-GLP-1(7-38);
Gly⁸Lys³⁴(N^{ε}-(lithocholoyl))-GLP-1(7-38); Gly⁸Lys^{26,34}-bis(N^{ε}-(lithocholoyl))-GLP-1(7-38);
Arg²⁶Lys³⁴(N^{ε}-(lithocholoyl))-GLP-1(7-38);
Gly⁸Arg²⁶Lys³⁴(N^{ε}-(choloyl))-GLP-1(7-39);
Lys²⁶(N^{ε}-(choloyl))Arg³⁴-GLP-1(7-39);
Gly⁸Lys²⁶(N^{ε}-(choloyl))Arg³⁴-GLP-1(7-39);
Arg^{26,34}Lys³⁶(N^{ε}-(choloyl))-GLP-1(7-39);
Gly⁸Arg^{26,34}Lys³⁶(N^{ε}-(choloyl))-GLP-1(7-39);
Lys²⁶(N^{ε}-(lithocholoyl))-GLP-1(7-39);
Lys³⁴(N^{ε}-(lithocholoyl))-GLP-1(7-39);
Lys^{26,34}-bis(N^{ε}-(lithocholoyl))-GLP-1(7-39);
Gly⁸Lys²⁶(N^{ε}-lithocholoyl))-GLP-1(7-39);
Gly⁸Lys³⁴(N^{ε}-(lithocholoyl))-GLP-1(7-39);
Gly⁸Lys^{26,34}-bis(N^{ε}-(lithocholoyl))-GLP-1(7-39);
Arg²⁶Lys³⁴(N^{ε}-(lithocholoyl))-GLP-1(7-39);
Gly⁸Arg²⁶Lys³⁴(N^{ε}-(choloyl))-GLP-1(7-40);
Lys²⁶(N^{ε}-(choloyl))Arg³⁴-GLP-1(7-40);
Gly⁸Lys²⁶(N^{ε}-(choloyl))Arg³⁴-GLP-1(7-40);
Arg^{26,34}Lys³⁶(N^{ε}-(choloyl))-GLP-1(7-40);
Gly⁸Arg^{26,34}Lys³⁶(N^{ε}-(choloyl))-GLP-1(7-40);
Lys²⁶(N^{ε}-(lithocholoyl))-GLP-1(7-40);
Lys³⁴(N^{ε}-(lithocholoyl))-GLP-1(7-40);
Lys^{26,34}-bis(N^{ε}-(lithocholoyl))-GLP-1(7-40);
Gly⁸Lys²⁶(N^{ε}-(lithocholoyl)-GLP-1(7-40);
Gly⁸Lys³⁴(N^{ε}-(lithocholoyl))-GLP-1(7-40);
Gly⁸Lys^{26,34}-bis(N^{ε}-(lithocholoyl))-GLP-1(7-40);
Arg²⁶Lys³⁴(N^{ε}-(lithocholoyl))-GLP-1(7-37);
Lys²⁶(N^{ε}-(lithocholoyl))-GLP-1(7-36);
Lys³⁴(N^{ε}-(lithocholoyl))-GLP-1(7-36);
Lys^{26,34}-bis(N^{ε}-(lithocholoyl))-GLP-1(7-36);
Gly⁸Lys²⁶(N^{ε}-(lithocholoyl))-GLP-1(7-36);
Gly⁸Lys³⁴(N^{ε}-(lithocholoyl))-GLP-1(7-36);
Gly⁸Lys^{26,34}-bis(N^{ε}-(lithocholoyl))-GLP-1(7-36);
Arg²⁶Lys³⁴(N^{ε}-(lithocholoyl))-GLP-1(7-36);
Lys²⁶(N^{ε}-(lithocholoyl))-GLP-1(7-35);
Lys³⁴(N^{ε}-(lithocholoyl))-GLP-1(7-35);
Lys^{26,34}-bis(N^{ε}-lithocholoyl))-GLP-1(7-35);
Gly⁸Lys²⁶(N^{ε}-(lithocholoyl))-GLP-1(7-35);
Gly⁸Lys³⁴(N^{ε}-(lithocholoyl))-GLP-1(7-35);
Gly⁸Lys^{26,34}-bis(N^{ε}-(lithocholoyl))-GLP-1(7-35);
Arg²⁶Lys³⁴(N^{ε}-(lithocholoyl))-GLP-1(7-35);
Lys²⁶(N^{ε}-(lithocholoyl))-GLP-1(7-36)amide;
Lys³⁴(N^{ε}-(lithocholoyl))-GLP-1(7-36)amide;
Lys^{26,34}-bis(N^{ε}-(lithocholoyl))-GLP-1(7-36)amide;
Gly⁸Lys²⁶(N^{ε}-(lithocholoyl))-GLP-1(7-36)amide;
Gly⁸Lys³⁴(N^{ε}-(lithocholoyl))-GLP-1(7-36)amide;
Gly⁸Lys^{26,34}-bis(N^{ε}-(lithocholoyl))-GLP-1(7-36)amide;
Arg²⁶Lys³⁴(N^{ε}-(lithocholoyl))-GLP-1(7-36)amide;
Gly⁸Arg²⁶Lys³⁴(N^{ε}-(lithocholoyl))-GLP-1(7-37);
Lys²⁶(N^{ε}-(lithocholoyl))Arg³⁴-GLP-1(7-37);
Gly⁸Lys²⁶(N^{ε}-(lithocholoyl))Arg³⁴-GLP-1(7-37);
Arg^{26,34}Lys³⁶(N^{ε}-(lithocholoyl))-GLP-1(7-37);
Arg^{26,34}Lys³⁸(N^{ε}-(lithocholoyl))-GLP-1(7-37);
Gly⁸Arg^{26,34}Lys³⁶(N^{ε}-(lithocholoyl))-GLP-1(7-37);
Gly⁸Arg²⁶Lys³⁴(N^{ε}-(lithocholoyl))-GLP-1(7-38);
Lys²⁶(N^{ε}-(lithocholoyl))Arg³⁴-GLP-1(7-38);
Gly⁸Lys²⁶(N^{ε}-(lithocholoyl))Arg³⁴-GLP-1(7-38);
Arg^{26,34}Lys³⁶(N^{ε}-(lithocholoyl))-GLP-1(7-38);
Arg^{26,34}Lys³⁸(N^{ε}-(lithocholoyl))-GLP-1(7-38);
Gly⁸Arg^{26,34}Lys³⁶(N^{ε}-(lithocholoyl))-GLP-1(7-38);
Gly⁸Arg²⁶Lys³⁴(N^{ε}-(lithocholoyl))-GLP-1(7-39);
Lys²⁶(N^{ε}-(lithocholoyl))Arg³⁴-GLP-1(7-39);
Gly⁸Lys²⁶(N^{ε}-(lithocholoyl))Arg³⁴-GLP-1(7-39);
Arg^{26,34}Lys³⁶(N^{ε}-(lithocholoyl))-GLP-1(7-39);
Gly⁸Arg^{26,34}Lys³⁶(N^{ε}-(lithocholoyl))-GLP-1(7-39);
Gly⁸Arg²⁶Lys³⁴(N^{ε}-(lithocholoyl))-GLP-1(7-40);
Lys²⁶(N^{ε}-(lithocholoyl))Arg³⁴-GLP-1(7-40);
Gly⁸Lys²⁶(N^{ε}-(lithocholoyl))Arg³⁴-GLP-1(7-40);
Arg^{26,34}Lys³⁶(N^{ε}-(lithocholoyl))-GLP-1(7-40); and
Gly⁸Arg^{26,34}Lys³⁶(N^{ε}-(lithocholoyl))-GLP-1(7-40). Each one of these specific GLP-1 derivatives constitutes an alternative embodiment of the invention..

The most preferred GLP-1. derivative is Arg³⁴, Lys²⁶(N^{ε}-(γ-Glu(N^{α}-hexadecanoyl)))-GLP-1(7-37).

In a further embodiment of the GLP-1 derivative, a parent peptide for a derivative of the invention is
Arg²⁶-GLP-1(7-37); Arg³⁴-GLP-1(7-37); Lys³⁶-GLP-1(7-37); Arg^{26,34}Lys³⁶-GLP-1(7-37);
Arg^{26,34}Lys³⁸GLP-1(7-38); Arg^{26,34}Lys³⁹-GLP-1(7-39); Arg^{26,34}Lys⁴⁰-GLP-1(7-40);
Arg²⁶Lys³⁶-GLP-1(7-37); Arg³⁴Lys³⁶-GLP-1(7-37); Arg²⁶Lys³⁹-GLP-1(7-39);
Arg³⁴Lys⁴⁰-GLP-1(7-40); Arg^{26,34}Lys^{36,39}-GLP-1(7-39); Arg^{26,34}Lys^{36,40}-GLP-1(7-40);
Gly⁸Arg²⁶-GLP-1(7-37); Gly⁸Arg³⁴-GLP-1(7-37); Gly⁸Lys³⁶-GLP-1(7-37);
Gly⁸Arg^{26,34}Lys³⁶-GLP-1(7-37); Gly⁸Azg^{26,34}Lys³⁹-GLP-1(7-39); Gly⁸Arg^{26,34}Lys⁴⁰-GLP-1(7-40); Gly⁸Arg²⁶Lys³⁶-GLP-1(7-37); Gly⁸Arg³⁴Lys³⁶-GLP-1(7-37); Gly⁸Arg²⁶Lys³⁹-GLP-1(7-39); Gly⁸Arg³⁴Lys⁴⁰-GLP-1(7-40); Gly⁸Arg^{26,34}Lys^{36,39}-GLP-1(7-39);
Gly⁸Arg^{26,34}Lys^{36,40}-GLP-1(7-10); Val⁸Arg²⁶-GLP-1(7-37); Val⁸Arg³⁴-GLP-1(7-37);
Val⁸Lys³⁶-GLP-1(7-37); Val⁸Arg^{26,34}Lys³⁶-GLP-1(7-37); Val⁸Arg^{26,34}Lys³⁹-GLP-1(7-39);
Val⁸Arg^{26,34}Lys⁴⁰-GLP-1(7-40); Val⁸Arg²⁶Lys³⁶-GLP-1(7-37); Val⁸Arg³⁴Lys³⁶-GLP-1(7-37); Val⁸Arg²⁶Lys³⁹-GLP-1(7-39); Val⁸Arg³⁴Lys⁴⁰-GLP-1(7-40); Val⁸Arg^{26,34}Lys^{36,39}-GLP-1(7-39); or Val⁸Arg^{26,34}Lys^{36,40}-GLP-1(7-40).

In a further embodiment, a parent peptide for a derivative of the invention is:.
Arg^{26,34}Lys³⁸GLP-1(7-38); Arg^{26,34}Lys³⁹GLP-1(7-39); Arg^{26,34}Lys⁴⁰GLP-1(7-40);
Arg^{26,34}Lys⁴¹GLP-1(7-41); Arg^{26,34}Lys⁴²GLP-1(7-42); Arg^{26,34}Lys⁴³GLP-1(7-43);
Arg^{26,34}Lys⁴⁴GLP-1(7-44); Arg^{26,34}Lys⁴⁵GLP-1(7-45); Arg²⁶Lys³⁸GLP-1(7-38);
Arg³⁴Lys³⁸GLP-1(7-38); Arg^{26,34}Lys^{36,38}GLP-1(7-38); Arg^{26,34}Lys³⁸GLP-1(7-38);
Arg²⁶Lys³⁹GLP-1(1-39); Arg³⁴Lys³⁹GLP-1(1-39); Arg^{26,34}Lys^{36,39}GLP-1(1-39);
Arg²⁶Lys³⁹GLP-1(7-39); Arg³⁴Lys³⁹GLP-1(7-39); Arg^{26,34}Lys^{36,39}GLP-1(7-39).

In a further embodiment, the present invention relates to a GLP-1 derivative wherein the parent peptide is selected from the group comprising Arg²⁶-GLP-1(7-37), Arg³⁴-GLP-1(7-37), Lys³⁶-GLP-1(7-37), Arg^{26,34}Lys³⁶-GLP-1(7-37), Arg²⁶Lys³⁶-GLP-1(7-37), Arg³⁴Lys³⁶-GLP-1(7-37), Gly⁸Arg²⁶-GLP-1(7-37), Gly⁸Arg³⁴-GLP-1(7-37), Gly⁸Lys³⁶-GLP-1(7-37), Gly⁸Arg^{26,34}Lys³⁶-GLP-1(7-37), Gly⁸Arg²⁶Lys³⁶-GLP-1(7-37) and Gly⁸Arg³⁴Lys³⁶-GLP-1(7-37).

In a further embodiment, the present invention relates to a GLP-1 derivative wherein the parent peptide is selected from the group comprising Arg²⁶Lys³⁸-GLP-1(7-38), Arg^{26,34}Lys³⁸-GLP-1(7-38), Arg^{26,34}Lys^{36,38}-GLP-1(7-38), Gly⁸Arg²⁶Lys³⁸-GLP-1(7-38) and Gly⁸Arg^{26,34}Lys^{36,38}-GLP-1(7-38).

In a further embodiment, the present invention relates to a GLP-1 derivative wherein the parent peptide is selected from the group comprising Arg²⁶Lys³⁹-GLP-1(7-39), Arg^{26,34}Lys^{36,39}-GLP-1(7-39), Gly⁸Arg²⁶Lys³⁹-GLP-1(7-39) and Gly⁸Arg^{26,34}Lys^{36,39}-GLP-1(7-39).

In a further embodiment, the present invention relates to a GLP-1 derivative wherein the parent peptide is selected from the group comprising Arg³⁴Lys⁴⁰-GLP-1(7-40), Arg^{26,34}Lys^{36,40}-GLP-1(7-40), Gly⁸Arg³⁴Lys⁴⁰-GLP-1(7-40) and Gly⁸Arg^{26,34}Lys^{36,40}-GLP-1(7-40).

In a further embodiment, the present invention relates to a GLP-1 derivative wherein the parent peptide is:
Arg²⁶-GLP-1(7-36); Arg³⁴-GLP-1(7-36); Arg^{26,34}Lys³⁶-GLP-1(7-36); Arg²⁶-GLP-1(7-36)amide; Arg³⁴-GLP-1(7-36)amide; Arg^{26,34}Lys³⁶-GLP-1(7-36)amide; Arg²⁶-GLP-1(7-37); Arg³⁴-GLP-1(7-37); Arg^{26,34}Lys³⁶-GLP-1(7-37); Arg²⁶-GLP-1(7-38); Arg³⁴-GLP-1(7-38); Arg^{26,34}Lys³⁸GLP-1(7-38); Arg²⁶-GLP-1(7-39); Arg³⁴-GLP-1(7-39); Arg^{26,34}Lys³⁹-GLP-1(7-39);
Gly⁸Arg²⁶-GLP-1(7-36); Gly⁸Arg³⁴-GLP-1(7-36); Gly⁸Arg^{26,34}Lys³⁶-GLP-1(7-36);
Gly⁸Arg²⁶-GLP-1(7-36)amide; Gly⁸Arg³⁴-GLP-1(7-36)amide; Gly⁸Arg^{26,34}Lys³⁶-GLP-1(7-36)amide; Gly⁸Arg²⁶-GLP-1(7-37); Gly⁸Arg³⁴-GLP-1(7-37); Gly⁸Arg^{26,34}Lys³⁶-GLP-1(7-37); Gly⁸Arg²⁶-GLP-1(7-38); Gly⁸Arg³⁴-GLP-1(7-38); Gly⁸Arg^{26,34}Lys³⁸GLP-1(7-38);
Gly⁸Arg²⁶-GLP-1(7-39); Gly⁸Arg³⁴-GLP-1(7-39); Gly⁸Arg^{26,34}Lys³⁹-GLP-1(7-39);
Val⁸Arg²⁶-GLP-1(7-36); Val⁸Arg³⁴-GLP-1(7-36); Val⁸Arg^{26,34}Lys³⁶-GLP-1(7-36);
Val⁸Arg²⁶-GLP-1(7-36)amide; Val⁸Arg³⁴-GLP-1(7-36)amide; Val⁸Arg^{26,34}Lys³⁶-GLP-1(7-36)amide; Val⁸Arg²⁶-GLP-1(7-37); Val⁸Arg³⁴-GLP-1(7-37); Val⁸Arg^{26,34}Lys³⁶-GLP-1(7-37); Val⁸Arg²⁶-GLP-1(7-38); Val⁸Arg³⁴-GLP-1(7-38); Val⁸Arg^{26,34}Lys³⁸GLP-1(7-38);
Val⁸Arg²⁶-GLP-1(7-39); Val⁸Arg³⁴-GLP-1(7-39); Val⁸Arg^{26,34}Lys³⁹-GLP-1(7-39);
Ser⁸Arg²⁶-GLP-1(7-36); Ser⁸Arg³⁴-GLP-1(7-36); Ser⁸Arg^{26,34}Lys³⁶-GLP-1(7-36);
Ser⁸Arg²⁶-GLP-1(7-36)amide; Ser⁸Arg³⁴-GLP-1(7-36)amide; Ser⁸Arg^{26,34}Lys³⁶-GLP-1(7-36)amide; Ser⁸Arg²⁶-GLP-1(7-37); Ser⁸Arg³⁴-GLP-1(7-37); Ser⁸Arg^{26,34}Lys³⁶-GLP-1(7-37); Ser⁸Arg²⁶-GLP-1(7-38); Ser⁸Arg³⁴-GLP-1(7-38); Ser⁸Arg^{26,34}Lys³⁸GLP-1(7-38);
Ser⁸Arg²⁶-GLP-1(7-39); Ser⁸Arg³⁴-GLP-1(7-39); Ser⁸Arg^{26,34}Lys³⁹-GLP-1(7-39);
Thr⁸Arg²⁶-GLP-1(7-36); Thr⁸Arg³⁴-GLP-1(7-36); Thr⁸Arg^{26,34}Lys³⁶-GLP-1(7-36);
Thr⁸Arg²⁶-GLP-1(7-36)amide; Thr⁸Arg³⁴-GLP-1(7-36)amide; Thr⁸Arg^{26,34}Lys³⁶-GLP-1(7-36)amide; Thr⁸Arg²⁶-GLP-1(7-37); Thr⁸Arg³⁴-GLP-1(7-37); Thr⁸Arg^{26,34}Lys³⁶-GLP-1(7-37); Thr⁸Arg²⁶-GLP-1(7-38); Thr⁸Arg³⁴-GLP-1(7-38); Thr⁸Arg^{26,34}Lys³⁸GLP-1(7-38);
Thr⁸Arg²⁶-GLP-1(7-39); Thr⁸Arg³⁴-GLP-1(7-39); Thr⁸Arg^{26,34}Lys³⁹-GLP-1(7-39);
Val⁸Glu³⁵Arg^{26,34}Lys³⁶-GLP-1(7-36); Val⁸Glu³⁵Arg^{26,34}Lys³⁶-GLP-1(7-36)amide;
Val⁸Glu³⁶Arg^{26,34}Lys³⁷GLP-1(7-37); Val⁸Glu³⁷Arg^{26,34}Lys³⁸GLP-1(7-38);
Val⁸Glu³⁸Arg^{26,34}Lys³⁹-GLP-1(7-39); Val⁸Glu³⁵Arg^{26,34}Lys³⁶-GLP-1(7-36);
Val⁸Glu³⁵Arg^{26,34}Lys³⁶-GLP-1(7-36)amide; Val⁸Glu³⁶Arg^{26,34}Lys³⁷GLP-1(7-37);
Val⁸Glu³⁷Arg^{26,34}Lys³⁸GLP-1(7-38); Val⁸Glu³⁸Arg^{26,34}Lys³⁹-GLP-1(7-39);
Val⁸Asp³⁵Arg^{26,34}Lys³⁶-GLP-1(7-36); Val⁸Asp³⁵Arg^{26,34}Lys³⁶-GLP-1(7-36)amide;
Val⁸Asp³⁶Arg^{26,34}Lys³⁷GLP-1(7-37); Val⁸Asp³⁷Arg^{26,34}Lys³⁸GLP-1(7-38);
Val⁸Asp³⁸Arg^{26,34}Lys³⁹-GLP-1(7-39); Val⁸Asp³⁵Arg^{26,34}Lys³⁶-GLP-1(7-36);
Val⁸Asp³⁵Arg^{26,34}Lys³⁶-GLP-1(7-36)amide; Val⁸Asp³⁶Arg^{26,34}Lys³⁷GLP-1(7-37);
Val⁸Asp³⁷Arg^{26,34}Lys³⁸GLP-1(7-38); Val⁸Asp³⁸Arg^{26,34}Lys³⁹-GLP-1(7-39);
Ser⁸Glu³⁵Arg^{26,34}Lys³⁶-GLP-1(7-36); Ser⁸Glu³⁵Arg^{26,34}Lys³⁶-GLP-1(7-36)amide;
Ser⁸Glu³⁶Arg^{26,34}Lys³⁷GLP-1(7-37); Ser⁸Glu³⁷Arg^{26,34}Lys³⁸GLP-1(7-38);
Ser⁸Glu³⁸Arg^{26,34}Lys³⁹-GLP-1(7-39); Ser⁸Glu³⁵Arg^{26,34}Lys³⁶-GLP-1(7-36);
Ser⁸Glu³⁵Arg^{26,34}Lys³⁶-GLP-1(7-36)amide; Ser⁸Glu³⁶Arg^{26,34}Lys³⁷GLP-1(7-37);
Ser⁸Glu³⁷Arg^{26,34}Lys³⁸GLP-1(7-38); Ser⁸Glu³⁸Arg^{26,34}Lys³⁹-GLP-1(7-39);
Ser⁸Asp³⁵Arg^{26,34}Lys³⁶-GLP-1(7-36); Ser⁸Asp³⁵Arg^{26,34}Lys³⁶-GLP-1(7-36)amide;
Ser⁸Asp³⁶Arg^{26,34}Lys³⁷GLP-1(7-37); Ser⁸Asp³⁷Arg^{26,34}Lys³⁸GLP-1(7-38);
Ser⁸Asp³⁸Arg^{26,34}Lys³⁹-GLP-1(7-39); Ser⁸Asp³⁵Arg^{26,34}Lys³⁶-GLP-1(7-36);
Ser⁸Asp³⁵Arg^{26,34}Lys³⁶-GLP-1(7-36)amide; Ser⁸Asp³⁶Arg^{26,34}Lys³⁷GLP-1(7-37);
Ser⁸Asp³⁷Arg^{26,34}Lys³⁸GLP-1(7-38); Ser⁸Asp³⁸Arg^{26,34}Lys³⁹-GLP-1(7-39);
Thr⁸Glu³⁵Arg^{26,34}Lys³⁶-GLP-1(7-36); Thr⁸Glu³⁵Arg^{26,34}Lys³⁶-GLP-1(7-36)amide;
Thr⁸Glu³⁶Arg^{26,34}Lys³⁷GLP-1(7-37); Thr⁸Glu³⁷Arg^{26,34}Lys³⁸GLP-1(7-38);
Thr⁸Glu³⁸Arg^{26,34}Lys³⁹-GLP-1(7-39); Thr⁸Glu³⁵Arg^{26,34}Lys³⁶-GLP-1(7-36);
Thr⁸Glu³⁵Arg^{26,34}Lys³⁶-GLP-1(7-36)amide; Thr⁸Glu³⁶Arg^{26,34}Lys³⁷GLP-1(7-37);
Thr⁸Glu³⁷Arg^{26,34}Lys³⁸GLP-1(7-38); Thr⁸Glu³⁸Arg^{26,34}Lys³⁹-GLP-1(7-39);
Thr⁸Asp³⁵Arg^{26,34}Lys³⁶-GLP-1(7-36); Thr⁸Asp³⁵Arg^{26,34}Lys³⁶-GLP-1(7-36)amide;
Thr⁸Asp³⁶Arg^{26,34}Lys³⁷GLP-1(7-37); Thr⁸Asp³⁷Arg^{26,34}Lys³⁸GLP-1(7-38);
Thr⁸Asp³⁸Arg^{26,34}Lys³⁹-GLP-1(7-39); Thr⁸Asp³⁵-Arg^{26,34}Lys³⁶-GLP-1(7-36);
Thr⁸Asp³⁵Arg^{26,34}Lys³⁶-GLP-1(7-36)amide; Thr⁸Asp³⁶Arg^{26,34}Lys³⁷GLP-1-(7-37);
Thr⁸Asp³⁷Arg^{26,34}Lys³⁸GLP-1(7-38); Thr⁸Asp³⁸Arg^{26,34}Lys³⁹-GLP-1(7-39);
Gly⁸Glu³⁵Arg^{26,34}Lys³⁶-GLP-1(7-36); Gly⁸Glu³⁵Arg^{26,34}Lys³⁶-GLP-1(7-36)amide;
Gly⁸Glu³⁶Arg^{26,34}Lys³⁷GLP-1(7-37); Gly⁸Glu³⁷Arg^{26,34}Lys³⁸GLP-1(7-38);
Gly⁸Glu³⁸Arg^{26,34}Lys³⁹-GLP-1(7-39); Gly⁸Glu³⁵Arg^{26,34}Lys³⁶-GLP-1(7-36);
Gly⁸Glu³⁵Arg^{26,34}Lys³⁶-GLP-1(7-36)amide, Gly⁸Glu³⁶Arg^{26,34}Lys³⁷GLP-1(7-37);
Gly⁸Glu³⁷Arg^{26,34}Lys³⁸GLP-1(7-38); Gly⁸Glu³⁸Arg^{26,34}Lys³⁹-GLP-1(7-39);
Gly⁸Asp³⁵Arg^{26,34}Lys³⁶-GLP-1(7-36); Gly⁸Asp³⁵Arg^{26,34}Lys³⁶-GLP-1(7-36)amide;
Gly⁸Asp³⁶Arg^{26,34}Lys³⁷GLP-1(7-37); Gly⁸Asp³⁷Arg^{26,34}Lys³⁸GLP-1(7-38);
Gly⁸Asp³⁸Arg^{26,34}Lys³⁹-GLP-1(7-39); Gly⁸Asp³⁵Arg^{26,34}Lys³⁶-GLP-1(7-36);
Gly⁸Asp³⁵Arg^{26,34}Lys³⁶-GLP-1(7-36)amide; Gly⁸Asp³⁶Arg^{26,34}Lys³⁷GLP-1(7-37);
Gly⁸Asp³⁷Arg^{26,34}Lys³⁸GLP-1(7-38); Gly⁸Asp³⁸Arg^{26,34}Lys³⁹-GLY-1(7-39);
Arg^{26,34}Lys¹⁸-GLP-1(7-36); Arg^{26,34}Lys¹⁸-GLP-1(7-36)amide; Arg^{26,34}Lys¹⁸GLP-1(7-37);
Arg^{26,34}Lys¹⁸GLP-1(7-38); Gly⁸Asp¹⁹Arg^{26,34}Lys¹⁸-GLP-1(7-36); Gly⁸Asp¹⁷Arg^{26,34}Lys¹⁸-GLP-1(7-36); Gly⁸Asp¹⁹Arg^{26,34}Lys¹⁸-GLP-1(7-36)amide; Gly⁸Asp¹⁷Arg^{26,34}Lys¹⁸-GLP-1(7-36)amide; Gly⁸Asp¹⁹Arg^{26,34}Lys¹⁸GLP-1(7-37); Gly⁸Asp¹⁹Arg^{26,34}Lys¹⁸GLP-1(7-38);
Gly⁸Asp¹⁷Arg^{26,34}Lys¹⁸GLP-1(7-38);
Arg^{26,34}Lys²³-GLP-1(7-36); Arg^{26,34}Lys²³-GLP-1(7-36)amide; Arg^{26,34}Lys²³GLP-1(7-37);
Arg^{26,34}Lys²³GLP-1(7-38); Gly⁸Asp²⁴Arg^{26,34}Lys²³-GLP-1(7-36); Gly⁸Asp²²Arg^{26,34}Lys²³-GLP-1(7-36); Gly⁸Asp²⁴Arg^{26,34}Lys²³-GLP-1(7-36)amide; Gly⁸Asp²²Arg^{26,34}Lys²³-GLP-1(7-36)amide; Gly⁸Asp²⁴Arg^{26,34}Lys²³GLP-1(7-37); Gly⁸Asp²⁴Arg^{26,34}Lys²³GLP-1(7-38);
Gly⁸Asp²²Arg^{26,34}Lys²³GLP-1(7-38);
Arg^{26,34}Lys²⁷-GLP-1(7-36); Arg^{26,34}Lys²⁷-GLP-1(7-36)amide; Arg^{26,34}Lys²⁷GLP-1(7-37);
Arg^{26,34}Lys²⁷GLP-1(7-38); Gly⁸Asp²⁸Arg^{26,34}Lys²⁷-GLP-1(7-36); Gly⁸Asp²⁶Arg^{26,34}Lys²⁷-GLP-1(7-36); Gly⁸Asp²⁸Arg^{26,34}Lys²⁷-GLP-1(7-36)amide; Gly⁸Asp²⁶Arg^{26,34}Lys²⁷-GLP-1(7-36)amide; Gly⁸Asp²⁸Arg^{26,34}Lys²⁷GLP-1(7-37); Gly⁸Asp²⁸Arg^{26,34}Lys²⁷GLP-1(7-38);
Gly⁸Asp²⁶Arg^{26,34}Lys²⁷GLP-1(7-38);
Arg^{26,34}Lys¹⁸-GLP-1(7-36); Arg^{26,34}Lys¹⁸-GLP-1(7-36)amide; Arg^{26,34}Lys¹⁸GLP-1(7-37);
Arg^{26,34}Lys¹⁸GLP-1(7-38); Val⁸Asp¹⁹Arg^{26,34}Lys¹⁸-GLP-1(7-36); Val⁸Asp¹⁷Arg^{26,34}Lys¹⁸-GLP-1(7-36); Val⁸Asp¹⁹Arg^{26,34}Lys¹⁸-GLP-1(7-36)amide; Val⁸Asp¹⁷Arg^{26,34}Lys¹⁸-GLP-1(7-36)amide; Val⁸Asp¹⁹Arg^{26,34}Lys¹⁸GLP-1(7-37); Val⁸Asp¹⁹Arg^{26,34}Lys¹⁸GLP-1(7-38);
Val⁸Asp¹⁷Arg^{26,34}Lys¹⁸GLP-1(7-38);
Arg^{26,34}Lys²³-GLP-1(7-36); Arg^{26,34}Lys²³-GLP-1(7-36)amide; Arg^{26,34}Lys²³GLP-1(7-37);
Arg^{26,34}Lys²³GLP-1(7-38); Val⁸Asp²⁴Arg^{26,34}Lys²³-GLP-1(7-36); Val⁸Asp²²Arg^{26,34}Lys²³-GLP-1(7-36); Val⁸Asp²⁴Arg^{26,34}Lys²³-GLP-1(7-36)amide; Val⁸Asp²²Arg^{26,34}Lys²³-GLP-1(7-36)amide; Val⁸Asp²⁴Arg^{26,34}Lys²³GLP-1(7-37); Val⁸Asp²⁴Arg^{26,34}Lys²³GLP-1(7-38);
Val⁸Asp²²Arg^{26,34}Lys²³GLP-1(7-38);
Arg^{26,34}Lys²⁷-GLP-1(7-36); Arg^{26,34}Lys-GLP-1(7-36)amide; Arg^{26,34}Lys²⁷GLP-1(7-37);
Arg^{26,34}Lys²⁷GLP-1(7-38); Val⁸Asp²⁸Arg^{26,34}Lys²⁷-GLP-1(7-36); Val⁸Asp²⁶Arg^{26,34}Lys²⁷-GLP-1(7-36); Val⁸Asp²⁸Arg^{26,34}Lys²⁷-GLP-1(7-36)amide; Val⁸Asp²⁶Arg^{26,34}Lys²⁷-GLP-1(7-36)amide; Val⁸Asp²⁸Arg^{26,34}Lys²⁷GLP-1(7-37); Val⁸Asp²⁸Arg^{26,34}Lys²⁷GLP-1(7-38);
Val⁸Asp²⁶Arg^{26,34}Lys²⁷GLP-1(7-38);
Arg^{26,34}Lys¹⁸-GLP-1(7-36); Arg^{26,34}Lys¹⁸-GLP-1(7-36)amide; Arg^{26,34}Lys¹⁸GLP-1(7-37);
Arg^{26,34}Lys¹⁸GLP-1(7-38); Ser⁸Asp¹⁹Arg^{26,34}Lys¹⁸-GLP-1(7-36); Ser⁸Asp¹⁷Arg^{26,34}Lys¹⁸-GLP-1(7-36); Ser⁸Asp¹⁹Arg^{26,34}Lys¹⁸-GLP-1(7-36)amide; Ser⁸Asp¹⁷Arg^{26,34}Lys¹⁸-GLP-1(7-36)amide; Ser⁸Asp¹⁹Arg^{26,34}Lys¹⁸GLP-1(7-37); Ser⁸Asp¹⁹Arg^{26,34}Lys¹⁸GLP-1(7-38);
Ser⁸Asp¹⁷Arg^{26,34}Lys¹⁸GLP-1(7-38);
Arg^{26,34}Lys²³-GLP-1(7-36); Arg^{26,34}Lys²³-GLP-1(7-36)amide; Arg^{26,34}Lys²³GLP-1(7-37);
Arg^{26,34}Lys²³GLP-1(7-38); Ser⁸Asp²⁴Arg^{26,34}Lys²³-GLP-1(7-36); Ser⁸Asp²²Arg^{26,34}Lys²³-GLP-1(7-36); Ser⁸Asp²⁴Arg^{26,34}Lys²³-GLP-1(7-36)amide; Ser⁸Asp²²Arg^{26,34}Lys²³-GLP-1(7-36)amide; Ser⁸Asp²⁴Arg^{26,34}Lys²³GLP-1(7-37); Ser⁸Asp²⁴Arg^{26,34}Lys²³GLP-1(7-38);
Ser⁸Asp²²Arg^{26,34}Lys²³GLP-1(7-38);
Arg^{26,34}Lys²⁷-GLP-1(7-36); Arg^{26,34}Lys²⁷-GLP-1(7-36)amide; Arg^{26,34}Lys²⁷GLP-1(7-37);
Arg^{26,34}Lys²⁷GLP-1(7-38); Ser⁸Asp²⁸Arg^{26,34}Lys²⁷-GLP-1(7-36); Ser⁸Asp²⁶Arg^{26,34}Lys²⁷-GLP-1(7-36); Ser⁸Asp²⁸Arg^{26,34}Lys²⁷-GLP-1(7-36)amide; Ser⁸Asp²⁶Arg^{26,34}Lys²⁷-GLP-1(7-36)amide; Ser⁸Asp²⁸Arg^{26,34}Lys²⁷GLP-1(7-37); Ser⁸Asp²⁸Arg^{26,34}Lys²⁷GLP-1(7-38);
Ser⁸Asp²⁶Arg^{26,34}Lys²⁷GLP-1(7-38);
Arg^{26,34}Lys¹⁸-GLP-1(7-36); Arg^{26,34}Lys¹⁸-GLP-1(7-36)amide; Arg^{26,34}Lys¹⁸GLP-1(7-37);
Arg^{26,34}Lys¹⁸GLP-1(7-38); Thr⁸Asp¹⁹Arg^{26,34}Lys¹⁸-GLP-1(7-36); Thr⁸Asp¹⁷Arg^{26,34}Lys¹⁸-GLP-1(7-36); Thr⁸Asp¹⁹Arg^{26,34}Lys¹⁸-GLP-1(7-36)amide; Thr⁸Asp¹⁷Arg^{26,34}Lys¹⁸-GLP-1(7-36)amide; Thr⁸Asp¹⁹Arg^{26,34}Lys¹⁸GLP-1(7-37); Thr⁸Asp¹⁹Arg^{26,34}Lys¹⁸GLP-1(7-38);
Thr⁸Asp¹⁷Arg^{26,34}Lys¹⁸GLP-1(7-38);
Arg^{26,34}Lys²³-GLP-1(7-36); Arg^{26,34}Lys²³-GLP-1(7-36)amide; Arg^{26,34}Lys²³GLP-1(7-37);
Arg^{26,34}Lys²³GLP-1(7-38); Thr⁸Asp²⁴Arg^{26,34}Lys²³-GLP-1(7-36); Thr⁸Asp²²Arg^{26,34}Lys²³-GLP-1(7-36); Thr⁸Asp²⁴Arg^{26,34}Lys²³-GLP-1(7-36)amide; Thr⁸Asp²²Arg^{26,34}Lys²³-GLP-1(7-36)amide; Thr⁸Asp²⁴Arg^{26,34}Lys²³GLP-1(7-37); Thr⁸Asp²⁴Arg^{26,34}Lys²³GLP-1(7-38);
Thr⁸Asp²²Arg^{26,34}Lys²³GLP-1(7-38);
Arg^{26,34}Lys²⁷-GLP-1(7-36); Arg^{26,34}Lys²⁷-GLP-1(7-36)amide; Arg^{26,34}Lys²⁷GLP-1(7-37);
Arg^{26,34}Lys²⁷GLP-1(7-38); Thr⁸Asp²⁸Arg^{26,34}Lys²⁷-GLP-1(7-36); Thr⁸Asp²⁶Arg^{26,34}Lys²⁷-GLP-1(7-36); Thr⁸Asp²⁸Arg^{26,34}Lys²⁷-GLP-1(7-36)amide; Thr⁸Asp²⁶Arg^{26,34}Lys²⁷-GLP-1(7-36)amide; Thr⁸Asp²⁸Arg^{26,34}Lys²⁷GLP-1(7-37); Thr⁸Asp²⁸Arg^{26,34}Lys²⁷GLP-1(7-38);
Thr⁸Asp²⁶Arg^{26,34}Lys²⁷GLP-1(7-38).

In a further embodiment, the present invention relates to a GLP-1 derivative wherein the parent peptide is:
Arg²⁶Lys³⁶-GLP-1(7-36); Arg³⁴Lys³⁶-GLP-1(7-36); Arg²⁶Lys³⁶-GLP-1(7-37); Arg³⁴Lys³⁶-GLP-1(7-37); Arg²⁶Lys³⁷-GLP-1(7-37); Arg³⁴Lys³⁷-GLP-1(7-37); Arg²⁶Lys³⁹-GLP-1(7-39);
Arg³⁴Lys³⁹-GLP-1(7-39); Arg^{26,34}Lys^{36,39}-GLP-1(7-39);
Arg²⁶Lys¹⁸-GLP-1(7-36); Arg³⁴Lys¹⁸-GLP-1(7-36); Arg²⁶Lys¹⁸GLP-1(7-37);
Arg³⁴Lys¹⁸GLP-1(7-37); Arg²⁶Lys¹⁸GLP-1(7-38); Arg³⁴Lys¹⁸GLP-1(7-38); Arg²⁶Lys¹⁸GLP-1(7-39); Arg³⁴Lys¹⁸GLP-1(7-39);
Arg²⁶Lys²³-GLP-1(7-36); Arg³⁴Lys²³-GLP-1(7-36); Arg²⁶Lys²³GLP-1(7-37);
Arg³⁴Lys²³GLP-1(7-37); Arg²⁶Lys²³GLP-1(7-38); Arg³⁴Lys²³GLP-1(7-38); Arg²⁶Lys²³GLP-1(7-39); Arg³⁴Lys²³GLP-1(7-39);
Arg²⁶Lys²⁷-GLP-1(7-36); Arg³⁴Lys²⁷-GLP-1(7-36); Arg²⁶Lys²⁷GLP-1(7-37);
Arg³⁴Lys²⁷GLP-1(7-37); Arg²⁶Lys²⁷GLP-1(7-38); Arg³⁴Lys²⁷GLP-1(7-38); Arg²⁶Lys²⁷GLP-1(7-39); Arg³⁴Lys²⁷GLP-1(7-39);
Arg^{26,34}Lys^{18,36}-GLP-1(7-36); Arg^{26,34}Lys¹⁸GLP-1(7-37); Arg^{26,34}Lys^{18,37}GLP-1(7-37);
Arg^{26,34}Lys^{18,38}GLP-1(7-38); Arg^{26,34}Lys^{18,39}GLP-1(7-39); Arg^{26,34}Lys^{23,36}-GLP-1(7-36);
Arg^{26,34}Lys²³GLP-1(7-37); Arg^{26,34}Lys^{23,37}GLP-1(7-37); Arg^{26,34}Lys^{23,38}GLP-1(7-38);
Arg^{26,34}Lys^{23,39}GLP-1(7-39); Arg^{26,34}Lys^{27,36}-GLP-1(7-36); Arg^{26,34}Lys²⁷GLP-1(7-37);
Arg^{26,34}Lys^{27,37}GLP-1(7-37); Arg^{26,34}Lys^{27,38}GLP-1(7-38); Arg^{26,34}Lys^{27,39}GLP-1(7-39);
Gly⁸GLP-1(7-36); Gly⁸GLP-1(7-37); Gly⁸GLP-1(7-38); Gly⁸GLP-1(7-39)
Gly⁸Arg²⁶Lys³⁶-GLP-1(7-36); Gly⁸Arg³⁴Lys³⁶-GLP-1(7-36); Gly⁸Arg²⁶Lys³⁶-GLP-1(7-37);
Gly⁸Arg³⁴Lys³⁶-GLP-1(7-37); Gly⁸Arg²⁶Lys³⁷-GLP-1(7-37); Gly⁸Arg³⁴Lys³⁷-GLP-1(7-37);
Gly⁸Alg²⁶Lys³⁹-GLP-1(7-39); Gly⁸Arg³⁴Lys³⁹-GLP-1(7-39); Gly⁸Arg^{26,34}Lys^{36,39}-GLP-1(7-39);
Gly⁸Arg²⁶Lys¹⁸-GLP-1(7-36); Gly⁸Arg³⁴Lys¹⁸-GLP-1(7-36); Gly⁸Arg²⁶Lys¹⁸GLP-1(7-37);
Gly⁸Arg³⁴Lys¹⁸GLP-1(7-37); Gly⁸Arg²⁶Lys¹⁸GLP-1(7-38); Gly⁸Arg³⁴Lys¹⁸GLP-1(7-38);
Gly⁸Arg²⁶Lys¹⁸GLP-1(7-39); Gly⁸Arg³⁴Lys¹⁸GLP-1(7-39);
Gly⁸Arg²⁶Lys²³-GLP-1(7-36); Gly⁸Arg³⁴Lys²³-GLP-1(7-36); Gly⁸Arg²⁶Lys²³GLP-1(7-37);
Gly⁸Arg³⁴Lys²³GLP-1(7-37); Gly⁸Arg²⁶Lys²³GLP-1(7-38); Gly⁸Arg³⁴Lys²³GLP-1(7-38);
Gly⁸Arg²⁶Lys²³GLP-1(7-39); Gly⁸Arg³⁴Lys²³GLP-1(7-39);
Gly⁸Arg²⁶Lys²⁷-GLP-1(7-36); Gly⁸Arg³⁴Lys²⁷-GLP-1(7-36); Gly⁸Arg²⁶Lys²⁷GLP-1(7-37);
Gly⁸Arg³⁴Lys²⁷GLP-1(7-37); Gly⁸Arg²⁶Lys²⁷GLP-1(7-38); Gly⁸Arg³⁴Lys²⁷GLP-1(7-38);
Gly⁸Arg²⁶Lys²⁷GLP-1(7-39); Gly⁸Arg³⁴Lys²⁷GLP-1(7-39);
Gly⁸Arg^{26,34}Lys^{18,36}-GLP-1(7-36); Gly⁸Arg^{26,34}Lys¹⁸GLP-1(7-37); Gly⁸Arg^{26,34}Lys^{18,37}GLP-1(7-37); Gly⁸Arg^{26,34}Lys^{18,38}GLP-1(7-38); Gly⁸Arg^{26,34}Lys^{18,39}GLP-1(7-39);
Gly⁸Arg^{26,34}Lys^{23,36}-GLP-1(7-36); Gly⁸Arg^{26,34}Lys²³GLP-1(7-37); Gly⁸Arg^{26,34}Lys^{23,37}GLP-1(7-37); Gly⁸Arg^{26,34}Lys^{23,38}GLP-1(7-38); Gly⁸Arg^{26,34}Lys^{23,39}GLP-1(7-39);
Gly⁸Arg^{26,34}Lys^{27,36}-GLP1(7-36); Gly⁸Arg^{26,34}Lys²⁷GLP-1(7-37); Gly⁸Arg^{26,34}Lys^{27,37}GLP-1(7-37); Gly⁸Arg^{26,34}Lys^{27,38}GLP-1(7-38); Gly⁸Arg^{26,34}Lys^{27,39}GLP-1(7-39);
Val⁸GLP-1(7-36); Val⁸GLP-1(7-37); Val⁸GLP-1(7-38); Val⁸GLP-1(7-39) Val⁸Arg²⁶Lys³⁶-GLP-1(7-36); Val⁸Arg³⁴Lys³⁶-GLP-1(7-36); Val⁸Arg²⁶Lys3⁶⁻GLP-1(7-37);
Val⁸Arg³⁴Lys³⁶-GLP-1(7-37); Val⁸Arg²⁶Lys³⁷-GLP-1(7-37); Val⁸Arg³⁴Lys³⁷-GLP-1(7-37);
Val⁸Arg²⁶Lys³⁹-GLP-1(7-39); Val⁸Arg³⁴Lys³⁹-GLP-1(7-39); Val⁸Arg^{26,34}Lys^{36,39}-GLP-1(7-39);
Val⁸Arg²⁶Lys¹⁸-GLP-1(7-36); Val⁸Arg³⁴Lys¹⁸-GLP-1(7-36); Val⁸Arg²⁶Lys¹⁸GLP-1(7-37);
Val⁸Arg³⁴Lys¹⁸GLP-1(7-37); Val⁸Arg²⁶Lys¹⁸GLP-1(7-38); Val⁸Arg³⁴Lys¹⁸GLP-1(7-38);
Val⁸Arg²⁶Lys¹⁸GLP-1(7-39); Val⁸Arg³⁴Lys¹⁸GLP-1(7-39);
Val⁸Arg²⁶Lys²³GLP-1(7-36); Val⁸Arg³⁴Lys²³-GLP-1(7-36); Val⁸Arg²⁶Lys²³GLP-1(7-37);
Val⁸Arg³⁴Lys²³GLP-1(7-37); Val⁸Arg²⁶Lys²³GLP-1(7-38); Val⁸Arg³⁴Lys²³GLP-1(7-38);
Val⁸Arg²⁶Lys²³GLP-1(7-39); Val⁸Arg³⁴Lys²³GLP-1(7-39);
Val⁸Arg²⁶Lys²⁷-GLP-1(7-36); Val⁸Arg³⁴Lys²⁷-GLP-1(7-36); Val⁸Arg²⁶Lys²⁷GLP-1(7-37);
Val⁸Arg³⁴Lys²⁷GLP-1(7-37); Val⁸Arg²⁶Lys²⁷GLP-1(7-38); Val⁸Arg³⁴Lys²⁷GLP-1(7-38);
Val⁸Arg²⁶Lys²⁷GLP-1(7-39); Val⁸Arg³⁴Lys²⁷GLP-1(7-39);
Val⁸Arg^{26,34}Lys^{18,36}-GLP-1(7-36); Val⁸Arg^{26,34}Lys¹⁸GLP-1(7-37); Val⁸Arg^{26,34}Lys^{18,37}GLP-1(7-37); Val⁸Arg^{26,34}Lys^{18,38}GLP-1(7-38); Val⁸Arg^{26,34}Lys^{18,39}GLP-1(7-39);
Val⁸Arg^{26,34}Lys^{23,36}-GLP-1(7-36); Val⁸Arg^{26,34}Lys²³GLP-1(7-37); Val⁸Arg^{26,34}Lys^{23,37}GLP-1(7-37); Val⁸Arg^{26,34}Lys^{23,38}GLP-1(7-38); Val⁸Arg^{26,34}Lys^{23,39}GLP-1(7-39);
Val⁸Arg^{26,34}Lys^{27,36}-GLP-1(7-36); Val⁸Arg^{26,34}Lys²⁷GLP-1(7-37); Val⁸Arg^{26,34}Lys^{27,37}GLP-1(7-37); Val⁸Arg^{26,34}Lys^{27,38}GLP-1(7-38); Val⁸Arg^{26,34}Lys^{27,39}GLP-1(7-39).

In a further embodiment GLP-1 or an analogue or a derivative thereof or a GLP-1 agonist is a GLP-1 agonist.

In a further embodiment the GLP-1 agonist is a molecule, preferably a non-peptide, which binds to a GLP-1 receptor with an affinity constant, K_{D}, below 1 µM, preferably below 100 nM.

In a further embodiment the GLP-1 agonist is selected from exendin as well as analogs, derivatives, and fragments thereof, preferably exendin-3 and -4.

Any possible combination of two or more of the embodiments described herein, is comprised within the scope of the present invention.

For a description of suitable compositions etc.. reference is made to WO 98/08871. (Novo Nordisk A/S).

Pharmaceutical compositions containing GLP-1 or an analogue or a derivative thereof or a GLP-1 agonist may be prepared by conventional techniques, *e.g*. as described in Remington's *Pharmaceutical Sciences,* 1985 or in Remington: *The Science and Practice of Pharmacy*, 19^{th} edition, 1995.

Thus, compositions of the GLP-1 or an analogue or a derivative thereof or a GLP-1. agonist can be prepared using the conventional techniques of the pharmaceutical industry which involves dissolving and mixing the ingredients as appropriate to give the desired end product.

According to one procedure, the GLP-1 or an analogue or a derivative thereof or a GLP-1 agonist is dissolved in an amount of water which is somewhat less than the final volume of the composition to be prepared. An isotonic agent, a preservative and a buffer is added as required and the pH value of the solution is adjusted - if necessary - using an acid, *e.g*. hydrochloric acid, or a base, *e.g*. aqueous sodium hydroxide as needed. Finally; the volume of the solution is adjusted with water to give the desired concentration of the ingredients.

Examples of isotonic agents are sodium chloride, mannitol and glycerol.

Examples of preservatives are phenol, m-cresol, methyl p-hydroxybenzoate and benzyl alcohol.

Examples of suitable buffers are sodium acetate and sodium phosphate.

Further to the above-mentioned components, solutions containing a GLP-1 or an analogue or a derivative thereof or a GLP-1 agonist may also contain a surfactant in order to improve the solubility and/or the stability of the GLP-1 or an analogue or a derivative thereof or a GLP-1 agonist.

A composition of certain peptides may, for example, be prepared as described in European Patent No. 272097 (to Novo Nordisk A/S) or in WO 93/18785.

According to one embodiment of the present invention, the GLP-1 or an analogue or a derivative thereof or a GLP-1 agonist is provided in the form of a composition. Such a composition can either be ready for use or it can be an amount of a solid composition, *e.g.* a lyophilised product. The solution preferably contains not less than about 2 mg/ml, preferably not less than about 5. mg/ml, more preferred not less than about 10 mg/ml of the GLP-1 or an analogue or a derivative thereof or a GLP-1 agonist and, preferably, not more than about 100 mg/ml of the GLP-1 or an analogue or a derivative thereof or a GLP-1 agonist.

### Example 1

The protocol was a slight modification of the procedure described by Billestrup and Nielsen (Billestrup N, Nielsen JH: The stimulatory effect of growth hormone, prolactin, and placental lactogen on beta cell proliferation is not mediated by insulin-like growth factor-I. Endocrinology 1991; 129:883-888.). Pancreatic islets were isolated from newborn rats by the collagenase method and cultured for 2-5 days before use. 2000 islets were transfered to 15 ml plastic tubes and washed once with Ca/Mg-free Hank's balanced salt solution. 500 µl cold trypsin solution (0.05% trypsin, 0.53 mM EDTA in Ca/Mg-free Hank's solution).. The islets were dispersed by aspiration with a pipette. 5 ml culture medium RPMI 1640 with 2% human serum was added. 75,000 islet cells were then placed in tissue culture flasks previously coated with ECL cell attachment matrix (Upstate Biotechnology) with 2 ml culture medium with 1 µg/ml human growth hormone (hGH) (Norditropin, Novo. Nordisk). After 7. days in culture at 37C the medium was replaced with culture medium without hGH or with addition of 100nM GLP-1, 5 µM Arg³⁴, Lys²⁶(N-ε-(γ-Glu(N-α-hexadecanoyl)))-GLP-1(7-37) or 200 ng/ml hGH. After 2 days in culture 10 µM 5-bromo-2-deoxyuridine. (BrdU) was added and after 90 min the medium was removed and the cells fixed in 1% paraformaldehyde in 0.1 M phosphate buffer. The cells were then stained with antibodies to. BrdU and insulin as described (Billestrup and Nielsen, 1991). The number of labelled beta cells in the absence of hormones was 0.6% and in the presence of hGH 3.5%. In the presence of GLP-1 the number was 1.7% and in the presence of Arg³⁴, Lys²⁶(N-ε-(γ-Glu(N-α-hexadecanoyl)))-GLP-1(7-37) 1.4%.

### Example 2

The male Zucker Diabetic Fatty fa/fa (ZDF) rat is a model of Type 2 diabetes. The rats are insulin resistant but normoglycemic from birth and they develop diabetes from about week 7 to week 10 of age. During the transitional period, the animals go through a state of impaired glucose tolerance. Although the animals are hyperinsulinemic before diabetes onset and during the early stages of diabetes, they later lose glucose-stimulated insulin secretion and finally become almost completely insulinopenic.

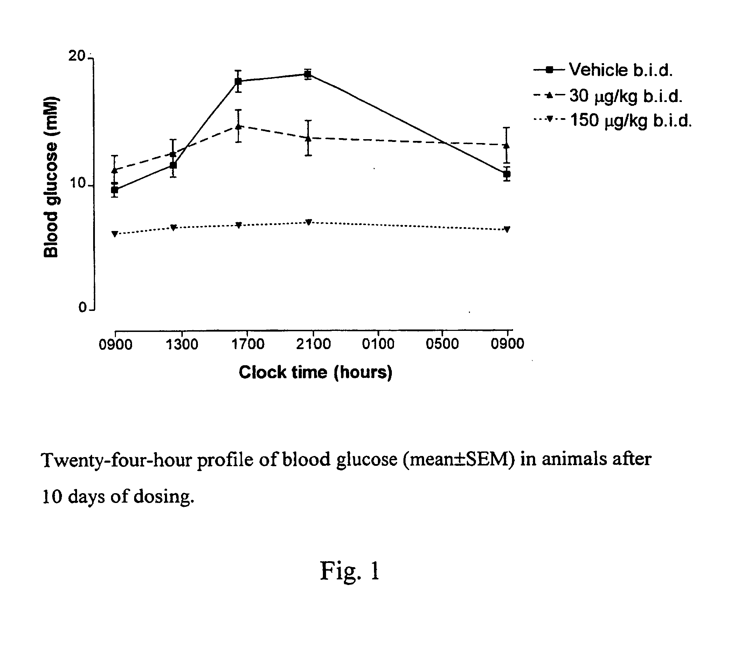

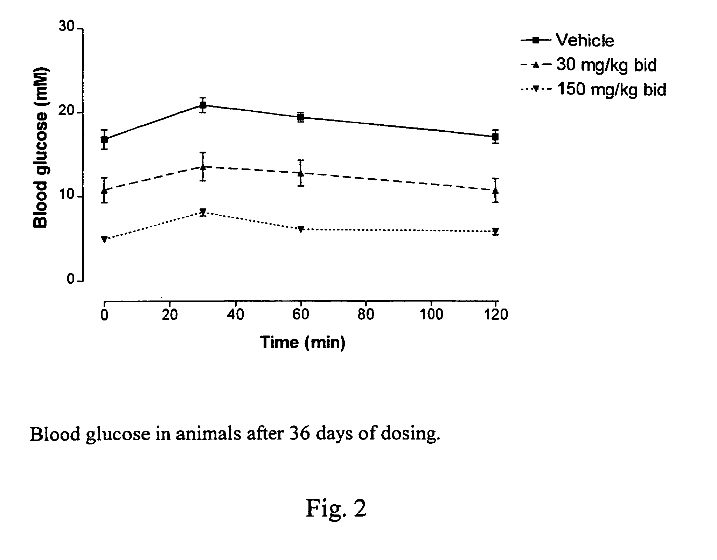

We have studied the effects of Arg³⁴, Lys²⁶(N-ε-(γ-Glu(N-α-hexadecanoyl)))-GLP-1(7-37) therapy during a period of time when the animals would normally progress from having impaired glucose tolerance to having overt Type 2 diabetes. Three groups of male ZDF rats (Genetic Models Inc, Indianapolis, Indiana, USA) were studied and dosed subcutaneously bi-daily with either vehicle (group A), 30 (group B) or 150 µg/kg (group C) of Arg³⁴, Lys²⁶(N-ε-(γ-Glu(N-α-hexadecanoyl)))-GLP-1(7-37), n=6 per group. Animals were between 7 and 8 weeks old when dosing was initiated, and fed glucose levels were not different between the groups before dosing began. However, they were elevated compared to a group of non-diabetic. Sprague-Dawley rats who had fed glucose levels significantly below the ZDF animals (6.4±0.6 vs 5.8±0.8, mean±SD, p<0.02). This demonstrates the relative impaired glucose tolerant state of the ZDF animals when the study began. After 10 days of dosing, group C had blood glucose levels during a normal 24-hour feeding schedule that were unchanged compared to the initial measurements and they were significantly lower than the vehicle- and low-dose-treated animals who were hyperglycemic. (Fig 1, p<0.0002 by ANOVA, total area under the curve used as summary measure). After 36 days of dosing, an oral glucose tolerance test was performed in the animals after an 11-hour fast. One g/kg of glucose was administered by oral gavage and subsequent measurements of blood glucose and plasma insulin were made. Also in this test, the glycemic level was significantly lower in group C compared to groups A and B (Fig 2 upper panel, p<0.0002 by ANOVA, total area under the curve used as summary measure. These results demonstrate that treatment with Arg³⁴, Lys²⁶(N-ε-(γ-Glu(N-α-hexadecanoyl)))-GLP-1(7-37) can prevent or delay the progression of impaired glucose tolerance to Type 2 diabetes.

### Example 3

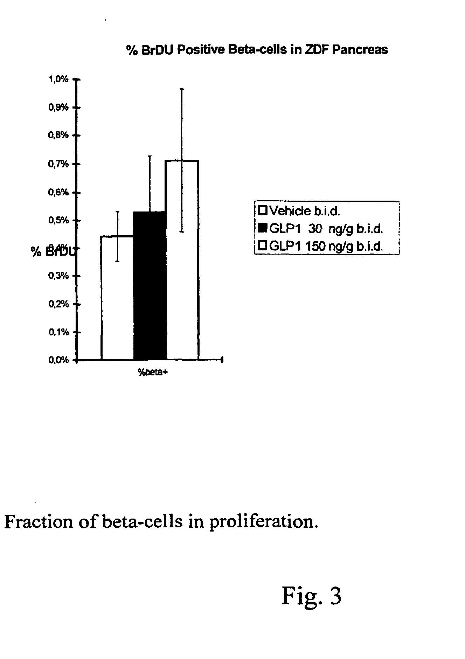

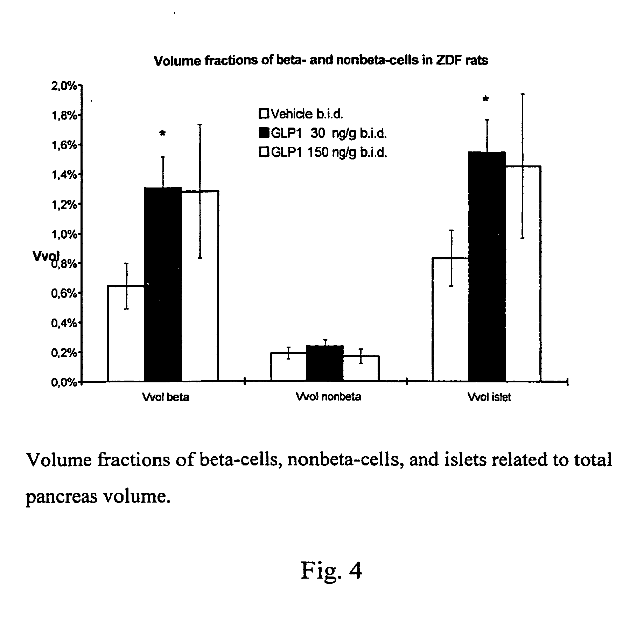

The rat experiment described in example 2 were examined for effects of Arg³⁴, Lys²⁶(N-ε-(γ-Glu(N-α-hexadecanoyl)))-GLP-1(7-37). (named GLP1 in fig. 3. and 4) on beta-cell growth and neogenesis.

Bromodeoxyuridine (BrDU) is incorporated in newly synthesized DNA and thus will label replicating cells. Six hours before sacrifice the rats were given an injection of 100 mg BrDU/kg intraperitoneally. After sacrifice the pancreata were fixed in 4% PFA, dehydrated, embedded in paraffin, and 3-4 mm sections double stained for BrDU and insulin for the measurement of beta-cell proliferation rate.

Insulin was stained with guinea pig anti-insulin, peroxidase-coupled rabbit anti-guinea pig Ig, and developed with AEC to give a red stain. BrDU was stained by monoclonal mouse anti-BrDU, biotinylated goat anti-mouse. Ig, avidin peroxidase, and developed with DAB and CuSO₄ to give a dark brown stain. BrDU stained nuclei of cells with insulin stained cytoplasm was examined in more than 1500 cells per section. The examination of the sections were carried out with the origin of the sections blinded to the observer. The rats treated with Arg³⁴, Lys²⁶(N-ε-(γ-Glu(N-α-hexadecanoyl)))-GLP-1(7-37) showed a dose dependent increase in the fraction of beta-cells that had incorporated BrDU as a result of stimulated cell proliferation.

Neighbor sections were stained for insulin and the combination of glucagonsomatostatin-pancreatic polypeptide for the measurement of the relative mass of islet beta-cells and nonbeta-cells. The beta-cells were stained for insulin as described above. The nonbeta-cells were stained with a mixture of monoclonal mouse anti-glucagon + rabbit anti-somatostatin + rabbit anti-pancreatic polypeptide, detected by biotinylated swine anti-multible Ig's, avidin peroxidase, and developed with DAB. and CuSO₄ to give a dark brown stain.. The volume fractions of beta- and nonbeta-cells were estimated by point counting stereologic techniques.

The beta-cell fraction of the total pancreas was significantly higher in the rats given Arg³⁴, Lys²⁶(N-ε-(γ-Glu(N-α-hexadecanoyl)))-GLP-1(7-37) at 30 ng/g for 6 weeks compared to vehicle treated rats, while there was no further increase in rats given doses of 150 ng/g.

## Claims

1. Use of GLP-1 or an analogue or a derivative thereof or a GLP-1 agonist for stimulation of beta-cell proliferation in vitro.

2. The use according to claim 1, wherein human growth hormone, a growth hormone releasing agent or a growth factor such as prolactin or placental lactogen is also used.

3. The use according to. any one of claims 1-2, wherein the GLP-1 or an analogue or a derivative thereof or a GLP-1 agonist is a GLP-1 derivative.

4. The use according to claim 3, wherein at least one amino acid residue of said GLP-1. derivative has a lipophilic substituent attached.

5. The use according to claim 4, wherein said GLP-1 derivative has a lipophilic substituent which is attached, optionally via a spacer, to the ε-amino group of a Lys residue.

6. The use according to claim 5, wherein said GLP-1 derivative is Arg³⁴, Lys²⁶(N-ε-(γ-Glu(N-α-hexadecanoyl)))-GLP-1(7-37).

7. The use according to any one of claims 1-2, wherein the GLP-1 or an analogue or a derivative thereof or a GLP-1 agonist is a GLP-1 analogue.

8. The use according to claim 7, wherein said GLP-1 analogue is selected from GLP-1(7-37) and GLP-1(7-36) amide and the corresponding Gly⁸ and Val⁸ analogues.

9. The use according to claim 7, wherein said GLP-1 analogue is selected from the Thr⁸ and Met⁸ analogues of GLP-1(7-37) and GLP-1(7-36) amide.

10. The use according to any one of claims 1-2, wherein the GLP-1 or an analogue or a derivative thereof or a GLP-1 agonist is a GLP-1 agonist..

11. The use according to claim 10, wherein said GLP-1 agonist is a molecule, preferably a non-peptide, which binds to. a GLP-1 receptor with an affinity constant, K_{D}, below 1 µM.

12. The use according to any one of claims 10-11, wherein said GLP-1 agonist is selected from exendin as well as analogs, derivatives, and fragments thereof, preferably exendin-3 and exendin-4.

## Patentansprüche

1. Verwendung von GLP-1 oder eines Analogs oder eines Derivats davon oder einem GLP-1 Agonist zur Stimulierung von Beta-Zellproliferation in vitro.

2. Verwendung nach Anspruch 1, wobei humanes Wachstumshormon, ein Wachstumshormon-freisetzender Wirkstoff oder ein Wachstumsfaktor, wie Prolactin oder Plazenta-Lactogen ebenfalls verwendet wird.

3. Verwendung nach einem beliebigen der Ansprüche 1-2, wobei das GLP-1 oder ein Analog oder ein Derivat davon oder ein GLP-1-Agonist ein GLP-1-Derivat ist.

4. Verwendung nach Anspruch 3, wobei mindestens ein Aminosäurerest des GLP-1-Derivats einen lipophilen Substituenten angeheftet besitzt.

5. Verwendung nach Anspruch 4, wobei das GLP-1-Derivat einen lipophilen Substituenten besitzt, welcher wahlweise über einen Spacer an die ε-Aminogruppe eines Lysinrestes angeheftet ist.

6. Verwendung nach Anspruch 5, wobei das GLP-1-Derivat Arg³⁴, Lys²⁶(N-ε-(γ-Glu(N-α-hexadecanoyl)))-GLP-1(7-37) ist.

7. Verwendung nach einem beliebigen der Ansprüche 1-2, wobei das GLP-1 oder ein Analog oder ein Derivat davon oder ein GLP-1-Agonist ein GLP-1-Analog ist.

8. Verwendung nach Anspruch 7, wobei das GLP-1-Analog ausgewählt ist aus GLP-1(7-37)- und GLP-1(7-36)-Amid und den entsprechenden Gly⁸- und Val⁸-Analoga.

9. Verwendung nach Anspruch 7, wobei das GLP-1-Analog ausgewählt ist aus den Thr⁸- und Met⁸-Analoga des GLP-1(7-37)- und GLP-1(7-36)-Amids.

10. Verwendung.nach einem beliebigen der Ansprüche 1-2, wobei das GLP-1 oder ein Analog oder ein Derivat davon oder ein GLP-1-Agonist ein GLP-1-Agonist ist.

11. Verwendung nach Anspruch 10, wobei der GLP-1-Agonist ein Molekül ist, bevorzugt ein Nicht-Peptid, welches an einen GLP-1-Rezeptor mit einer Affinitätskonstante, K_{D}, unterhalb von 1 µM bindet.

12. Verwendung nach einem beliebigen der Ansprüche 10-11, wobei der GLP-1-Agonist ausgewählt ist aus sowohl Exendin als auch Analoga, Derivaten und Fragmenten davon, bevorzugt Exendin-3 und Exendin-4.

## Revendications

1. Utilisation de GLP-1 ou d'un analogue ou d'un dérivé de celui-ci ou d'un agoniste de GLP-1 pour une stimulation de prolifération de cellules bêta in vitro.

2. Utilisation selon la revendication 1, dans laquelle une hormone de croissance humaine, un agent de libération d'hormone de croissance ou un facteur de croissance tel que la prolactine ou un lactogène placentaire, est également utilisé.

3. Utilisation selon l'une quelconque des revendications 1 à 2, dans laquelle le GLP-1 ou un analogue ou un dérivé de celui-ci ou un agoniste de GLP-1 est un dérivé de GLP-1.

4. Utilisation selon la revendication 3, dans laquelle au moins un résidu d'acide aminé dudit dérivé de GLP-1 a un substituant lipophile fixé.

5. Utilisation selon la revendication 4, dans laquelle ledit dérivé de GLP-1 a un substituant lipophile qui est fixé, de manière facultative via un élément d'espacement, au groupe ε-aminé d'un résidu Lys.

6. Utilisation selon la revendication 5, dans laquelle ledit dérivé de GLP-1 et Arg³⁴, Lys²⁶ (N-E- (γ-Glu(N-α-hexadécanoyle)))-GLP-1(7-37).

7. Utilisation selon l'une quelconque des revendications 1 à 2, dans laquelle le GLP-1 ou un analogue ou un dérivé de celui-ci ou un agoniste de GLP-1 est un analogue de GLP-1.

8. Utilisation selon la revendication 7, dans laquelle ledit analogue de GLP-1 est sélectionné parmi un amide GLP-1(7-37) et GLP-1(7-36) et les analogues Gly⁸ et Val⁸ correspondants.

9. Utilisation selon la revendication 7, dans laquelle ledit analogue de GLP-1 est sélectionné parmi les analogues Thr⁸ et Met⁸ d'un amide GLP-1(7-37) et GLP-1(7-36).

10. Utilisation selon l'une quelconque des revendications 1 à 2, dans laquelle le GLP-1 ou un analogue ou un dérivé de celui-ci ou un agoniste de GLP-1 est un agoniste de GLP-1.

11. Utilisation selon la revendication 10, dans laquelle ledit agoniste de GLP-1 est une molécule, de préférence non-peptidique, qui se lie à un récepteur de GLP-1 ayant une constante d'affinité, K_{D}, inférieure à 1 µM.

12. Utilisation selon l'une quelconque des revendications 10 à 11, dans laquelle ledit agoniste de GLP-1 est sélectionné parmi l'exendine ainsi que des analogues, dérivés, et fragments de celle-ci, de préférence exendine-3 et exendine-4.
